(19) Europäisches Patentamt / European Patent Office / Office européen des brevets

(11) **EP 4 438 707 A1**

(12) **EUROPEAN PATENT APPLICATION**
published in accordance with Art. 153(4) EPC

(43) Date of publication:
**02.10.2024  Bulletin 2024/40**

(21) Application number: **22899171.7**

(22) Date of filing: **22.02.2022**

(51) International Patent Classification (IPC):
*C11D 3/386* (2006.01)      *C11D 1/66* (2006.01)
*C11D 1/04* (2006.01)      *C11D 1/29* (2006.01)
*C11D 1/83* (2006.01)      *C11D 1/90* (2006.01)
*C11D 3/37* (2006.01)      *C11D 3/43* (2006.01)
*C11D 3/20* (2006.01)      *C11D 17/04* (2006.01)
*C12N 9/14* (2006.01)

(52) Cooperative Patent Classification (CPC):
**C11D 1/04; C11D 1/29; C11D 1/66; C11D 1/83;
C11D 1/90; C11D 3/20; C11D 3/37; C11D 3/386;
C11D 3/43; C11D 17/04; C12N 9/14**

(86) International application number:
**PCT/RU2022/000054**

(87) International publication number:
**WO 2023/096523 (01.06.2023 Gazette 2023/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **24.11.2021  RU 2021134314**

(71) Applicant: **SkyLab AG
Zurich, 8001 (CH)**

(72) Inventors:
• **BELOUS, Elena Yur'evna
  Moscow,  121309 (RU)**
• **FILATOV, Viktor Andreyevich
  Chaykovskiy, 617762 (RU)**

(74) Representative: **Glawe, Delfs, Moll
Partnerschaft mbB von
Patent- und Rechtsanwälten
Postfach 13 03 91
20103 Hamburg (DE)**

(54) **LIPASE-BASED BIODEGRADABLE DETERGENT COMPOSITION**

(57)    The present invention relates to a biodegradable lipase-based detergent composition for cleavage of low-melting acylglycerides of different spatial configurations and control of foam stability and hydrophilization of surfaces. The composition is intended to be included in detergents use of which enables control of hydrophilization of surfaces and foam stability upon application of different surfaces and also enables control of kinetics of enzymatic cleavage of low-melting saturated and/or unsaturated acids and their monoacyl-, diacyl- and triacylglycerides with different numbers of bonds with sp$^2$-hy-bridization having cis- or trans-configuration.

EP 4 438 707 A1

**Description**

FIELD OF INVENTION

The present invention relates to a biodegradable lipase-based detergent composition for cleavage of low-melting acylglycerides of different spatial configurations and control of foam stability and hydrophilization of surfaces. The composition is intended for inclusion in household chemicals use of which enables control of hydrophilization of surfaces and foam stability upon application on different surfaces and it also makes it possible to control kinetics of enzymatic cleavage of low-melting saturated and/or unsaturated acids and their monoacyl-, diacyl- and triacylglycerides with different numbers of bonds with sp$^2$-hybridization having cis- or trans-configuration. Chemical bonds with sp$^2$-hybridization are preferably represented by bonds in a hydrocarbon chain from 4 to 22 carbon atoms in $\omega 3y$ positions, where y takes a value from 1 to 7. The composition is safe for hand skin and can be used for producing household chemicals for sensitive hand skin such as laundry detergents, dish-washing detergents, floor cleaners, glass cleaners, all-purpose detergents and others.

BACKGROUND OF INVENTION

Development of household chemicals with improved consumer properties remains a promising direction in the category of household products. The volume of the global market of washing and cleaning products will reach 170 billion $ in 2021 and will increase to 207 billion $ by 2026, the forecasted growth will make up 21.8% during 5 years [The Future of Sustainable Cleaning Products to 2026, Smithers]. According to experts, in Great Britain alone by the end of 2021 the category of cleaning products will grow by 9% CAGR and will make up £485 million comparing to 2019 [Dishwashing products: Impact of COVID-19, UK, July 2020, Mintel], while in China the market volume increases by 4% CAGR annually and will make up RMB17,195 million by 2024 [Dishwashing Products - China - February 2020, Mintel]. In RF alone in 2020, despite controversial expectations, the volume of production of laundry and household detergents made up ~2 million tons, demonstrating the growth of +1.05% due to cleaning products and increase of the average per capita consumption of laundry detergents. Based on the volumes of the internal market of Russia, average per capita consumption in 2020 made up 11.1 kg of household chemicals per capita annually, therefore the trend for development of biodegradable and effective products will remain ["Bytovaya Khimiya" ("Household Chemicals"), information and analytical journal No. 78-79, December 2020-March 2021].

According to the data of Mintel analytical database, over 51% of consumers in Great Britain as a highly developed market of household chemicals are worried that biodegradable and natural products are not so effective as common ones [A year of innovation in Household cleaners, April 2021, Mintel], while 48% of consumers reduced use of natural cleaners in favor of common petrochemical-based household chemicals. Consumers point out importance of development of natural biodegradable household chemicals as they support sustainable development and production initiatives.

Consumers from different regions of the world seek natural, effective and biodegradable products that will contain biodegradable substances, will not harm the environment and autonomous septic tanks in private residences, while remaining effective against fat and stubborn soils. For example, 38% of consumers in Germany and Italy seek cleaners for hard surfaces that are biodegradable [Promote expertise to appeal to homecare consumers, 25 May 2021, Mintel]. Highly developed markets of EU - Germany, France, Poland - are interested in innovation, natural, biodegradable surface cleaners. According to Mintel GNPD analyses, launch of all-purpose cleaners with "eco-friendly" and "biodegradable" claims on labels increased, on average, from 54% to 67% (+13%) during 5 years in the territory of EU. For example, in Poland, France and Germany the number of such launches during 2020 made up 70%, 65% and 86%, respectively, which demonstrates the growing trend for ecological and environmentally safe household chemicals. The given trend can be observed not only in EMEA, but also in APAC and North America. According to the survey of 2000 consumers of Mintel/Dynata, over 46% of consumers in Thailand stated that products with natural biodegradable ingredients are safer than chemical alternatives. More than 54% of consumers of Canada (25-44 years old), according to their own beliefs, prefer use of natural surface cleaners.

Every day people use quite a number (>5) of household chemicals: laundry detergents, auxiliary laundry detergents, cleaning and washing products, bleachers, etc. More often consumers pay attention to functional characteristics of products of their choice, particularly, effectiveness of removing domestic soils (types of soils, difficulty of their removal, rate of removal), additional effects, for example, odor control at home, safety for cleaned surfaces and hand skin, as well as eco-friendliness and possibility of use in residences with autonomous sewage systems and septic tanks. Based on the Lightspeed/Mintel survey in March 2019, over 38% of consumers in Great Britain tend to choose multi-functional household chemicals enabling reducing the time required for full home cleanup. According to the data of the analytical survey Dishwashing products: Impact of COVID-19, UK, July 2020 Mintel, by 2020-2025 the trend for sustainable products, with a high content of natural components, a long "free from" list, as well as presence of biodegradable components will grow. It should be emphasized that consumption of household chemicals in EAEU and EU countries does not depend on the income level of population as they are essential goods. However, according to the results of the Lightspeed/Mintel survey in March 2020, 10-16% of consumers aged 18 years and older started to use household chemicals less often because of low effectiveness (10% of the surveyed people), a high content of synthetic chemical substances and low biodegradability of products (11% of the surveyed people), high water consumption for removal of soils (36% of the surveyed people). In addition, attractiveness and possibility of purchase of household chemicals in EMEA region were assessed. The main stimulating factors are reduction of physical efforts for manual removal of soils (more than 33% of responses), reduction of water consumption for washing away soils (more than 28% of responses) and high proven effectiveness of the product (more than 27% of responses). Thus, a need in development of innovation household chemicals that enable effective removal of domestic soils, reduce required efforts for surface cleaning and are biodegradable in the environment was revealed.

However, despite the demand for effective products the consumer still pays attention to dermatological comfort for hand skin after use of cleaning and washing household products. Every day people touch a large number of surfaces in public places and at home and use household chemicals. To protect oneself against development of dermatological diseases, it is necessary to carefully select household chemicals as it is dermatological comfort of hands that is a guarantee of health and, as a result, high quality of people's life. Due to frequent contact of hands with synthetic components in products, development of products with a high content of natural ingredients remains the most reliable way of maintaining skin health and, as a result, its healthy appearance. According to specialists' estimates, skin health is one of the basics of general human health. For example, 2% of sodium lauryl sulfate, known as SLS, in the composition of household products can cause loss of transepidermal moisture by 68.9 g/m2/h in relation to the normal level in 12 hours after contacting the product [Loffler, H., & Happle, R. (2003). Profile of irritant patch testing with detergents: sodium lauryl sulfate, sodium laureth sulfate and alkyl polyglucoside. Contact Dermatitis, 48(1), 26-32]. As the content of sodium lauryl sulfate in household chemicals can reach 29% in connection with a high washing capacity, this can negatively affect the hand skin condition. In addition, petrochemical surfactants cause keratin denaturation by destruction of sulfide bonds and formation of sulfhydryl groups -SH on human cells and epidermal barrier washout [Prottey C, Ferguson T. Factors which determine the skin irritation potential of soaps and detergents. J Soc Cosmet Chem. 1975;26: 29-46.]. It was revealed that aggressive anionic surfactants sodium decyl sulfate (SDS), sodium myristyl sulfate (SMS), sodium tridecyl sulfate (STS) cause washout of water-soluble epidermis proteins by 166.1%, 163.9% and 198.5%, respectively [Loffler, H., & Happle, R. (2003). Profile of irritant patch testing with detergents: sodium lauryl sulfate, sodium laureth sulfate and alkyl polyglucoside. Contact Dermatitis, 48(1), 26-32]. Thus, development of formulations of cleaning and washing products with biodegradable surfactants for effective removal of low-melting acylglycerides of saturated and/or unsaturated acids with different numbers of bonds with $sp^2$-hybridization having cis- or trans-configuration is a high-priority direction for ensuring dermatological comfort of hand skin and environmental protection.

PRIOR ART

Search for effective components and their combinations as means for removal of household soils of different origin, in particular, lipid soils, is a daily task of companies producing cleaning and washing products. The main components of household chemicals are surfactants, fillers, components providing for stability of formulations, and functional additives performing specific functions. In particular, an example of special functional additives is biolipase.

Lipase, or acylglycerol-acyl-hydrolase (enzyme classification code E.C.3.1.1.3, CAS 9001-62-1, EINECS 232-619-9) is a water-soluble enzyme of the class of hydrolases that catalyses hydrolysis of ester bonds in triglycerides of fatty acids being water-insoluble esters of glycerol and higher carboxylic acids of different structures and stereochemical configurations [European Commission Cosmetic Ingredients & Substances Database: http://ec.europa.eu/growth/tools-databases/cosing/].

Lipase is a catalyst of the reaction of cleavage of low-melting acylglycerides of saturated and/or unsaturated acids with different numbers of bonds with $sp^2$-hybridization having cys- or trans-configuration and is not a product/starting substance in the hydrolysis reaction, which enables improving kinetics of the enzymatic reaction. 1 enzyme molecule is known to be capable of catalysing up to 10000 reactions per second depending on domain organization, functional activity and crude materials. A small enzyme quantity is enough for effective action as activity of commercially available lipases is above 40 U/g or U/ml. For example, an effective concentration of lipase is 0.4-0.8% (w/w) in complex additives to be included in household chemicals [https://www.enzymeinnovation.com/lipase-detergent-everything-you-need-know/].

Lipases are believed to be one on the most effective enzymes for removal of fat stains and lipid soils based on vegetable oils (sunflower, olive, rapeseed, corn, flax-seed oil and others) having different fatty-acid formulations, animal fats (butter, lard, beef tallow, mutton tallow, etc.), having a solid aggregate state in normal conditions, lubricating materials based on ester components and greasy phases of perfumes and cosmetics containing oils, waxes, emulsifiers with ester bonds such as lecithin, perfume components and essential oils. As these stains are insoluble in aqueous phase and can be removed only with micellar solutions of surfactants with limited solubility, lipase contributes to hydrolysis of ester bonds and destruction of substrates, because of which surfactants of certain origin remove surface soils better, penetrate deep into fabric and more slowly reach saturation of micelles while the washing effectiveness remains.

Lipases have a high activity in relation to lipid soils on different surfaces, including metal, polymeric, wooden, ceramic ones, and do not destroy the structure of these surfaces, which speaks of gentle action in formulations of washing products. The most desirable pH-optimum makes up from 5.0 to 11.0, while the activity can remain at different temperatures from 20°C to 90°C, specifically the temperature optimum makes up from 20°C to 60°C. Lipases can be stable in presence of proteases, chelating agents, peroxide compounds (hydrogen peroxide, sodium percarbonate, etc.) and surfactants, specifically, anionic ones. Lipase stabilizers, specifically, glycerol, propylene glycols, sorbitol, sugars, carboxylic acids, alkylamines, inorganic salts, non-ionic and ionic surfactants can be included in the systems, and water content in the formulation can be reduced, which enables producing a concentrated washing product with low water consumption in production of detergents. Use of lipase makes it possible to produce energy-efficient products saving electric power and water and thus to use resources in a responsible manner.

Lipase concentration in household chemicals depends on activity and can make up from 0.0025 to 1% (by weight, in pure form). It was established that addition of lipase with activity 100 KLU3/g to surfactants in the formulation of a laundry detergent demonstrated high effectiveness in removal of fat stains during standard washing at 40°C. The effective lipase concentration (in complex additives) with this activity is 0.2-0.6% [H. Uhlig, E. M. Linsmaier-Bednar. Industrial enzymes and their applications. Engineering, April 1998, 472 pages. ISBN: 978-0-471-19660-0. DOI:10.5860/choice.36-0333]. In detergents lipase with activity of 50 U/mL in the amount of 10 mL together with 0.5% system of anionic and non-ionic surfactants was stable in the formulation and did not influence thermal stability of detergents. Also, lipase was not destroyed in presence of 2% hydrogen peroxide solution, i.e. it retained its activity by 92% in 2 hours after addition of a bleaching agent to the solution, however the activity significantly decreased in presence of sodium hypochlorite and sodium perborate with increase of the concentration from 1 to 2%.

It was pointed out that lipase has the higher activity in presence of non-ionic surfactants, specifically, the ones based on glucosides, than anionic surfactants. It was revealed that addition of lipase to 0.5% solution of non-ionic surfactants, on average, increases effectiveness of removal of fat stains and lipid soils by 10% according to results of laboratory tests when the following experimental conditions are kept: lipase activity 30 U/ml, wash temperature 37°C, wash duration 30 minutes. It should be noted that increasing of lipase concentration from 40 U/ml to 100 U/ml did not result in significant increase of effectiveness, which makes it possible to use the functional components in formulations efficiently and take care of the environment. Presence of 0.5% of non-ionic surfactants in the lipase solution results in greater removal of lipid and oily stains from 26% to 55%, the maximum effectiveness was achieved at 40°C and it made up 62%. From the point of view of effectiveness, the optimal time of lipase action in the wash process is 45 minutes [Mamta Chauhan, Rajinder Singh Chauhan, and Vijay Kumar Garlapati. Evaluation of a New Lipase from Staphylococcus sp. for Detergent Additive Capability. BioMed Research International Volume 2013 |Article ID 374967 | 6 pages | https://doi.org/10.1155/2013/374967. https://www.hindawi.com/journals/bmri/2013/374967/].

It was revealed that lipase is a necessary component in detergents for effective removal of lipid stains and fat soils. It is enzymatic activity per unit volume that determines the working concentration of the component in formulations of detergents of different applications. With time-based activity of 50 U/mL and in the amount of 10 mL, the total enzyme activity will make up 500 U. With average enzyme activity of 100-1000 U/g, the effective lipase concentration will make up 0.05-5.00% in detergent formulation [Mamta Chauhan, Rajinder Singh Chauhan, and Vijay Kumar Garlapati. Evaluation of a New Lipase from Staphylococcus sp. for Detergent Additive Capability. BioMed Research International Volume 2013 |Article ID 374967 | 6 pages| https://doi.org/10.1155/2013/374967. https://www.hindawi.com/journals/bmri/2013/374967/].

The study by Hemachander et al. evaluated effectiveness of removal of lipids by means of lipase with enzymatic activity of 40 U/mL. They applied olive oil on fabric pieces and waited till drying of stains by fixation of low-melting mono-, di- and triacylglycerides of saturated and/or unsaturated acids with different numbers of bonds with $sp2$-hybridization having cis- or trans-configuration on the surface. Effectiveness of removal of stains was evaluated by the gravimetric method enabling accurate evaluation of mass removal of soiling in grams. It should be noted that most surfactants and chelating agents increased lipase effectiveness by 5-10 U/mL, with the exception of sodium dodecyl sulfate (SDS). Lipase stability also remained in presence of surfactants, with the exception of sodium dodecyl sulfate (SDS), in presence of which activity of lipase decreased by 15 U/mL owing to destabilizing activity of the surfactant on the quarternary protein structure as a result of a high charge and ionic strength of the solution. Lipase itself increased effectiveness of removal of lipid stains by 30% in complete absence of surfactants in formulations. Addition of 100 U of lipase to 1% solution of surfactants in formulations of household chemicals increased effectiveness of the system by 15%, in total, and removal of fat stains made up to 65% of the original soiling. Increasing of lipase concentration above 100 U did not result in increase of activity due to kinetic reaction peculiarities, namely reaction levelling off because of enzyme saturation with the substrate. The best temperature for removal of lipid soils was from 30 to 50°C, while the optimum time interval was 30 minutes after the start of action [Hemachander, C., & Puvanakrishnan, R. (2000). Lipase from Ralstonia pickettii as an additive in laundry detergent formulations. Process Biochemistry, 35(8), 809-814.

doi:10.1016/s0032-9592(99)00140-5].

Thus, lipase is a necessary component in detergents for effective removal of lipid stains and fat soils due to targeted action on molecules of triglycerides of fatty acids and increase of effectiveness of detergents based on surfactants, specifically, non-ionic surfactants. With the average activity of the component of 100-10000 LCLU(DL)/g, the effective concentration of the component is 0.01-1% in cleaning and washing products for cleaning different surfaces.

A serious problem in home or industrial kitchens is removal of molecules of low-melting lipids and separation of volatile compounds that are perceived as strong odors, especially those emitted during frying food on vegetable oils and fats. As fats and oils contain esters of fatty acids and glycerol, the complex based on specific lipase enzyme and biodegradable surfactants with the function of hydrotropes improves the wash process by targeted cleavage of molecules of fats and absorption of released fatty acids. This synergetic action improves the washing capacity of household chemicals for different surfaces by control of kinetics of cleavage of low-melting saturated and/or unsaturated acids and their monoacyl-, diacyl- and triacylglycerides with different numbers of bonds with $sp^2$-hybridization having cis- or trans-configuration.

In the patent RU2294756C1 [CJSC "Petrospirt"] published on 10.03.2007 a liquid detergent is described that contains a mixture of ethoxylated C9-C15 alcohols 12.5-23.5% and alkyl polyglucoside 2.5-7.0% as a surfactant and additionally contains lipase 0.6-2.1% and other substances. The invention enables increasing effectiveness of pre-sterilization cleaning of medical products.

The above-described composition according to the patent RU2294756C1 has a number of disadvantages, namely, it contains a low-foaming surfactant in the form of a mixture of ethoxylated C9-C15 alcohols of synthetic origin, which limits the field of use to detergents for hard surfaces. The liquid detergent formulation contains from 35 to 65% of substances of natural origin (ISO 16128), therefore it cannot be called natural. Pre-sterilization cleaning of medical products and endoscopes is enabled primarily by presence of antiseptic components, specifically, benzyl alcohol, methylchloroisothiozolynone, methylisothiozolynone restricting growth of microorganisms, however it does not make it possible to fully remove biofilms of microorganisms in catheters and endoscopes in connection with a high accumulation of DNA molecules and absence of special substances for their cleavage.

The international patent application WO2017082961A1 [AMERICAN STERILIZER COMPANY] published on 18.05.2017 discloses the formulation and application of a washing and desinfecting composition consisting of two parts. The desinfecting component is peracetic acid, hydrogen peroxide, sulfuric acid or their mixture and the washing nonenzymatic substance is alkanolamine, ethoxylated alcohol, alkylglucoside, alkylene glycol, alkyl dipropionate, alkyl dialkylamine oxide or a mixture of 2 or more substances. The composition can be supplemented by enzymes, for example, amylase, lipase, carbohydrase and protease or a mixture thereof. The invention makes it possible to increase effectiveness of cleaning of medical products, specifically, endoscopes.

The above-described composition according to WO2017082961A1 contains different groups of surfactants free of sulfates and having low or moderate foaming properties. There are auxiliary substances, specifically, EDTA, borates, phosphonates and their derivatives (aminotrimethyl, hydroxyethylidene), carboxymethylinulin, methylglycinediacetate, having the ability of accumulating in the environment and prohibited by voluntary certification Vitality Leaf, Ecolabel. Replacement of these components with natural biodegradable alternatives would enable producing a biodegradable formula, obtaining an ecocertificate of Level I and increase loyalty of consumers all over the world.

In distinction to RU2294756C1 and WO2017082961A1, the authors of the present invention studied effectiveness of the composition containing biolipase and an aerobically readily biodegradable surfactant and established the synergetic effect for hydophilization of different surfaces and foam stability during application on different surfaces as well as control of kinetics of enzymatic cleavage of low-melting saturated and/or unsaturated acids and their monoacyl-, diacyl- and triacylglycerides with different numbers of bonds with $sp^2$-hybridization having cis- or trans-configuration. The composition made it possible, in a quick and targeted manner, to cleave complex lipid soils based on low-melting mono-, di- or triacylglycerides of unsaturated aliphatic acids with high omega-oxidation affinity and deposits from different surfaces (wooden, metal, enameled, polymeric, ceramic, tin-glazed and other ones), types of fabric (cotton, synthetic materials) in different temperature ranges and in different conditions. The combination is safe for hand skin and ensures dermatological comfort of skin.

The technical result of the innovation biodegradable composition consists in effective removal of complex lipid soils by control of hydrophilization of surfaces and foam stability upon application on different surfaces, as well as kinetics of enzymatic cleavage of low-melting saturated and/or unsaturated acids and their monoacyl-, diacyl- and triacylglycerides with different numbers of bonds with $sp^2$-hybridization having cis- or trans-configuration. The complex is active within a wide pH range, specifically, 5.0-12.0 units, and a wide temperature range, specifically, from +5 to +60°C, which extends the field of use in washing and cleaning products.

It was revealed that the combination of lipase and aerobically readily biodegradable surfactants enables increasing effectiveness of removal of mixed soils comprising the lipid component on different surfaces, specifically, on metal, polymeric, glass and wooden surfaces, as well as increasing washing effectiveness of products on different surfaces with the same content of surfactants based on components of natural origin and proven biodegradability of the formula according to the guideline 301OECD.

The combination of components makes it possible to achieve synergetic action as regards hydrophilization of surfaces, foam height control and to maintain effectiveness with a lower percentage of addition of certain components. Lipase obtained by modern biotechnology methods without use of GMO is an active enzyme cleaving insoluble lipid substrates with ester bonds of triglycerides on "water-oil" or "oil-water" phase boundary. Biodegradable surfactants based on glucosides act as co-activators (colipases) by binding with lipase domains and formation of a clathrate layer of water molecules, contributing to changing of conformation of the active center of lipase and reduction of process activation energy ($\Delta E_a$), which simplifies enzymatic cleavage of low-melting saturated and/or unsaturated acids and their monoacyl-, diacyl- and triacylglycerides with different numbers of bonds with $sp^2$-hybridization having cis- or trans-configuration. The lipase enzyme reduces activation energy by increasing the number of activated molecules of mono-, di- and triacylglycerides of aliphatic acids with a number of atoms from 4 to 22 and glycerol that become reactive on a lower energy level, which reduces the energy barrier for further enzymatic reaction. In addition, surfactants based on glucosides thanks to a hydrophilic surface owing to free hydroxyl groups -OH, specifically orient lipase substrates on "water-oil" or "oil-water" phase boundary, increasing availability of an ester bond for enzymatic hydrolysis. As the enzymatic reaction is an equilibrium reaction, displacing equilibrium towards formation of hydrolysis products requires binding free fatty acids and removing them from the system. Specifically, biodegradable surfactants based on glucosides and alkyl and/or alkyl sulfate and/or alkyl polyethylene glycol sulfate bind reaction products, displacing reaction equilibrium towards cleavage of lipid substrates in the system.

An additional property is increasing of surface or fabric wettability by hydrotropic properties of molecules of biodegradable surfactants, increasing of foam stability and foam height of the composition for better removal of oily and lipid-containing soils. Hydrophilization of surfaces and reduction of the Young contact angle ensure better flowability of the product over the surface, emulsifying of lipid-containing soils and reduction of soil redeposition. Low-melting saturated and/or unsaturated acids and their monoacyl-, diacyl- and triacylglycerides with different numbers of bonds with sp2-hybridization having cis- or trans-configuration in sunflower, olive, soybean, rapeseed oils have hydrophobic properties and are oxidized in air with formation of resinlike elastic films. Biodegradable surfactants based on glucosides provide for high hydrophilization of surfaces for solubilization and emulsification of solidifying acylglycerides, while alkyl and/or alkyl sulfate and/or alkyl polyethylene glycol sulfates bind released molecules of saturated and/or unsaturated acids with different numbers of bonds with sp2-hybridization having cis- and trans-configuration for maintaining and/or increasing foam stability and foam height.

The innovation composition comprising biolipase and biodegradable surfactants is intended for effective cleaning and removal of fat soils by controlling kinetics of the reaction of cleavage of lipid substrates, increasing hydrophilization of surfaces and foam stability of household chemicals. The formulation works in 3 stages: hydrophilization, penetration and enzymatic cleavage. Aerobically readily biodegradable surfactants wetten the contaminated surface and improve access of lipase to low-melting saturated and/or unsaturated acids and their monoacyl-, diacyl- and triacylglycerides with different numbers of bonds with sp2-hybridization that form an impenetrable oxidizable film on different surfaces and fabrics. Then biolipase deeply penetrates through the oily film and starts enzymatic cleavage, while biodegradable surfactants bind reaction products into the general system solution and prevent saturation, allosteric inhibition of the enzyme. The complex based on natural and biodegradable components is effective within the pH range of 5.0-12.0 units in presence of different natural components, which enables its application in a wide range of household chemicals for cleaning of different surfaces such as laundry and dishwashing detergents, floor and glass cleaners, all-purpose detergents. The components have targeted action on lipid and complex soils on different surfaces and also wetten the surface and leave no streaks after application. After use of the composition the cleanliness of surfaces and a pleasant scent last longer. Thus, combined use of the components in the claimed composition in one product results in increasing of kinetics of the enzymatic reaction of cleavage of complex household lipid soils by hydrophilization, stabilization, orientation of substrates and increasing of activity of lipase due to biodegradable surfactants, which makes it possible to do cleaning quickly even using cold water. A distinctive feature is the fact that the components in claimed concentrations act only towards complex soils and do not impair the look of most surfaces, specifically, metal, wooden, polymeric and enameled ones. The composition does not include substances made from petrochemicals and organic solvents, filming agents and alcohols, therefore washing and cleaning products with this composition can be used regularly with no harm to hand skin. Combined use of these components has synergetic action ensuring complex care of different surfaces by means of a single detergent for daily use. In addition, household chemicals based on this composition have a high percentage of natural ingredients, meet requirements of Ecolabel and Vitality Life voluntary ecocertification and pass aerobic biodegradability tests according to 301 OECD.

SUMMARY

In the first aspect the invention relates to a composition intended for use in a detergent comprising:

(A) Biolipase obtained by the biotechnology method from a microorganism, where the given biolipase has biological activity over 40 LU/g, pH 4.0-7.0 at 25°C; and

(B) An aerobically readily biodegradable surfactant or a mixture of surfactants selected from alkylglucoside with the general formula: $R^1$-O-$[G]p^1$, where $R^1$ is an alkyl and/or alkenyl group with hydrocarbon chain length from 4 to 22 carbon atoms, G is a saccharide fragment of 5 or 6 carbon atoms, $p^1$ takes a value from 1 to 4;

(C) An aerobically readily biodegradable surfactant or a mixture of surfactants selected from alkylcarboxylate, and/or alkyl sulfate, and/or alkyl polyethylene glycol sulfate with the general formula $R^2$-(O-$CH_2$-$CH_2$-O)$n^1$($SO_3$)$n^2$($CO_2$)$n^3X^1$, where $n^1$ takes a value from 0 to 10 and denotes the number of polyethylene groups, $R^2$ is an alkyl and/or alkenyl group with hydrocarbon chain length from 5 to 22 carbon atoms; $n^2$ takes a value from 0 to 1 and denotes the number of sulfate groups; $n^3$ takes a value from 0 to 1 and denotes the number of carboxyl groups; $X^1$ is an alkaline and/or alkaline earth metal, ammonium, alkylammonium, alkanolammonium, glucoammonium, basic aminoacid cation,

wherein the mass ratio of A, B and C components makes up (0.0003-0.25):(1-25):(0.5-25), respectively.

The composition of the present invention can additionally comprise an anionic surfactant, wherein the anionic surfactant is selected from:

- A salt of a higher fatty acid amide and methylglycine with the general formula $R^3$-C(O)-N(-$CH_3$)-$CH_2$-$CO_2X^2$, where $R^3$ is an alkyl and/or alkenyl group with hydrocarbon chain length from 5 to 21 carbon atoms and $X^2$ is an alkaline and/or alkaline earth metal, ammonium, alkylammonium, alkanolammonium, glucoammonium cation;
- Alkyl polyethylene glycol carboxylate with the general formula: $R^4$-O-(-$CH_2$-$CH_2$-O-)$n^4CH_2$-$CO_2X^3$, where $n^4$ takes a value from 1 to 15 and denotes the number of polyethylene glycol groups, $R^4$ is an alkyl and/or alkenyl group with hydrocarbon chain length from 6 to 22 carbon atoms and $X^3$ is an alkaline and/or alkaline earth metal, ammonium, alkylammonium, alkanolammonium, glucoammonium cation;
- A disubstituted salt of 2-sulfocarboxylic acid with the general formula: $R^5$-CH(-$SO_3X^4$)-$CO_2X^4$, where $R^5$ is an alkyl and/or alkenyl group with hydrocarbon chain length from 4 to 20 carbon atoms and $X^4$ is an alkaline and/or alkaline earth metal, ammonium, alkylammonium, alkanolammonium, glucoammonium cation;
- A mono- or disubstituted salt of a higher carboxylic acid amide and glutamic acid with the general formula: $R^6$-C(O)-NH-CH(-$CH_2$-$CH_2$-$CO_2X^5$)-$CO_2X^5$, where $R^6$ is an alkyl and/or alkenyl group with hydrocarbon chain length from 5 to 21 carbon atoms and $X^5$ is an alkaline and/or alkaline earth metal, ammonium, alkylammonium, alkanolammonium, glucoammonium or hydrogen cation;
- An salt of a higher fatty acid amide and glycine with the general formula: $R^7$-C(O)-NH-$CH_2$-$CO_2X^6$, where $R^7$ is an alkyl and/or alkenyl group with hydrocarbon chain length from 5 to 21 carbon atoms and $X^6$ is an alkaline and/or alkaline earth metal, ammonium, alkylammonium, alkanolammonium, glucoammonium cation;
- A salt of a higher fatty acid amide and alanine with the general formula: $R^8$-C(O)-NH-CH(-$CH_3$)-$CO_2X^7$, where $R^8$ is an alkyl and/or alkenyl group with hydrocarbon chain length from 5 to 21 carbon atoms and $X^7$ is an alkaline and/or alkaline earth metal, ammonium, alkylammonium, alkanolammonium, glucoammonium cation;
- A salt of a higher fatty acid amide and 2-aminomethylethanesulfonic acid with the general formula: $R^9$-C(O)-N(-$CH_3$)-$CH_2$-$CH_2$-$SO_3X^8$, where $R^9$ is an alkyl and/or alkenyl group with hydrocarbon chain length from 5 to 21 carbon atoms and $X^8$ is an alkaline and/or alkaline earth metal, ammonium, alkylammonium, alkanolammonium, glucoammonium cation;
- Alkylpolyglucoside hydroxypropylsulfonate with the general formula: $R^{10}$-O-$[G]p^2$-O-$CH_2$-CH(-OH)-$CH_2$-$SO_3X^9$, where $R^{10}$ is an alkyl and/or alkenyl group with hydrocarbon chain length from 6 to 22 carbon atoms, G is a saccharide fragment comprising 5 or 6 carbon atoms, $p^2$ takes a value from 1 to 4 and $X^9$ is an alkaline and/or alkaline earth metal, ammonium, alkylammonium, alkanolammonium, glucoammonium cation;
- Alkylpolyglucoside carboxylate with the general formula: $R^{11}$-O-$[G]p^3$-O-$CH_2$-$CO_2X^{10}$, where $R^{11}$ is an alkyl and/or alkenyl group with hydrocarbon chain length from 6 to 22 carbon atoms, G is a saccharide fragment comprising 5 or 6 carbon atoms, $p^3$ takes a value from 1 to 4 and $X^{10}$ is an alkaline and/or alkaline earth metal, ammonium, alkylammonium, alkanolammonium, glucoammonium cation;
- A salt of a higher fatty acid amide and threonine with the general formula: $R^{12}$-C(O)-NH-CH(-CH(-OH)-$CH_3$)-$CO_2X^{11}$, where $R^{12}$ is an alkyl and/or alkenyl group with hydrocarbon chain length from 5 to 21 carbon atoms and $X^{11}$ is an alkaline and/or alkaline earth metal, ammonium, alkylammonium, alkanolammonium, glucoammonium cation;
- A salt of a higher fatty acid amide and an aminoacid obtained by hydrolysis of proteins from plant raw materials with the general formula: $R^{13}$-C(O)-$AAX^{12}$, where $R^{13}$ is an alkyl and/or alkenyl group with hydrocarbon chain length from 5 to 21 carbon atoms, AA is an aminoacid or peptide obtained during hydrolysis of plant protein and $X^{12}$ is an alkaline and/or alkaline earth metal, ammonium, alkylammonium, alkanolammonium, glucoammonium cation;

or mixtures thereof.

The composition of the present invention can additionally comprise an amphoteric surfactant, where the amphoteric surfactant is selected from:

- A disubstituted salt of acylamphodiacetate with the general formula: $R^{14}$-C(O)-NH-$CH_2$-$CH_2$-N(-$CH_2$-$CO_2X^{13}$)-$CH_2$-$CH_2$-O-$CH_2$-$CO_2X^{13}$, where $R^{14}$ is an alkyl and/or alkenyl group with hydrocarbon chain length from 5 to 21 carbon atoms and $X^{13}$ is an alkaline and/or alkaline earth metal, ammonium, alkylammonium, alkanolammonium, glucoammonium cation;
- An acylamphoacetate salt with the general formula: $R^{15}$-C(O)-NH-$CH_2$-$CH_2$-N(-$CH_2$-$CO_2X^{14}$)-$CH_2$-$CH_2$-OH, where $R^{15}$ is an alkyl and/or alkenyl group with hydrocarbon chain length from 5 to 21 carbon atoms and $X^{14}$ is an alkaline and/or alkaline earth metal, ammonium, alkylammonium, alkanolammonium, glucoammonium cation;
- An alkylamphoacetate salt with the general formula: $R^{16}$-C(=N-$CH_2$-$CH_2$-N((-$CH_2$-$CH_2$-OH)-$CH_2$-$CO_2X^{15}$)-), where $R^{16}$ is an alkyl and/or alkenyl group with hydrocarbon chain length from 5 to 21 carbon atoms and $X^{15}$ is an alkaline and/or alkaline earth metal, ammonium, alkylammonium, alkanolammonium, glucoammonium cation;
- Acylamidoalkylbetaine with the general formula: $R^{17}$-C(O)-NH-$R^{18}$-N((-$CH_3$)$_2$)-$CH_2$-$CO_2$ , where $R^{17}$ is an alkyl and/or alkenyl group with hydrocarbon chain length from 5 to 21 carbon atoms and $R^{18}$ is an alkyl group with hydrocarbon chain length from 1 to 4 carbon atoms;
- Acylamidoalkylhydroxysultaine with the general formula: $R^{19}$-C(O)-NH-$R^{20}$-N(-$CH_3$)$_2$-$CH_2$-CH(-OH)-$CH_2$-$SO_3$, where $R^{19}$ is an alkyl and/or alkenyl group with hydrocarbon chain length from 5 to 21 carbon atoms and $R^{20}$ is an alkyl group with hydrocarbon chain length from 1 to 4 carbon atoms;
- Acylamidoalkylamine oxide with the general formula: $R^{21}$-C(O)-NH-$R^{22}$-N(-$CH_3$)$_2$-O , where $R^{21}$ is an alkyl and/or alkenyl group with hydrocarbon chain length from 5 to 21 carbon atoms and $R^{22}$ is an alkyl group with hydrocarbon chain length from 1 to 4 carbon atoms;
- Alkylbetaine with the general formula: $R^{23}$-N((-$CH_3$)$_2$)-$CH_2$-$CO_2$, where $R^{23}$ is an alkyl and/or alkenyl group with hydrocarbon chain length from 5 to 21 carbon atoms;
- Alkylhydroxysulfate with the general formula: $R^{24}$-N(-$CH_3$)$_2$-$CH_2$-CH(-OH)-$CH_2$-$SO_3$, where $R^{24}$ is an alkyl and/or alkenyl group with hydrocarbon chain length from 6 to 22 carbon atoms;
- Alkylsulfate with the general formula: $R^{25}$-N(-$CH_3$)$_2$-$CH_2$-$CH_2$-$CH_2$-$SO_3$, where $R^{25}$ is an alkyl and/or alkenyl group with hydrocarbon chain length from 6 to 22 carbon atoms;
- Alkylamine oxide with the general formula: $R^{26}$-N(-$CH_3$)$_2$-O, where $R^{26}$ is an alkyl and/or alkenyl group with hydrocarbon chain length from 6 to 22 carbon atoms;

or mixtures thereof.

The composition of the present invention can additionally comprise a non-ionic surfactant, where the non-ionic surfactant is selected from:

- Alkylpolyethyleneglycol with the general formula: $R^{27}$-O(-$CH_2$-$CH_2$-O-)$n^5$H, where $n^5$ takes a value from 2 to 20 and denotes the number of polyethyleneglycol groups and $R^{27}$ is an alkyl and/or alkenyl group with hydrocarbon chain length from 6 to 22 carbon atoms;
- Alkylpolyethylene/propyleneglycol with the general formula: $R^{28}$-O(-$CH_2$-$CH_2$-O-)$n^6$(-CH(-$CH_3$)-$CH_2$-O-)$n^7$H, where $n^6$ takes a value from 2 to 20 and denotes the number of polyethyleneglycol groups, $n^7$ takes a value from 2 to 20 and denotes the number of polypropyleneglycol groups and $R^{28}$ is an alkyl and/or alkenyl group with hydrocarbon chain length from 6 to 22 carbon atoms;
- Dialkylpolyethyleneglycol with the general formula: $R^{29}$-O(-$CH_2$-$CH_2$-O-)$n^8R^{30}$, where $n^8$ takes a value from 2 to 20 and denotes the number of polyethyleneglycol groups, $R^{29}$ is an alkyl and/or alkenyl group with hydrocarbon chain length from 6 to 22 carbon atoms and $R^{30}$ is an alkyl and/or alkenyl group with hydrocarbon chain length from 1 to 12 carbon atoms;
- Dialkylpolyethylene/propyleneglycol with the general formula: $R^{31}$-O(-$CH_2$-$CH_2$-O-)$n^9$(-CH(-$CH_3$)-$CH_2$-O-)$n^{10}$-$R^{32}$, where $n^9$ takes a value from 2 to 20 and denotes the number of polyethyleneglycol groups, $n^{10}$ takes a value from 2 to 20 and denotes the number of polypropyleneglycol groups , $R^{31}$ is an alkyl and/or alkenyl group with hydrocarbon chain length from 6 to 22 carbon atoms and $R^{32}$ is an alkyl and/or alkenyl group with hydrocarbon chain length from 1 to 12 carbon atoms;

or mixtures thereof.

The composition of the present invention can additionally comprise a dispersion medium for a polysaccharide/solvent, where the dispersion medium for a polysaccharide/solvent is selected from:

- An organic alcohol with the general formula: $R^{33}$(-OH)$s^1$, where $R^{33}$ is an alkyl group with hydrocarbon chain length from 3 to 12 carbon atoms, $s^1$ takes a value from 1 to 12 and denotes the number of hydroxyl groups arranged in the hydrocarbon radical in any order with respect to each other;

- Alkylpolypropyleneglycol with the general formula: $H(-CH(-CH_3)-CH_2-O-)n^{11}R^{34}$, where $n^{11}$ takes a value from 2 to 10 and denotes the number of polypropyleneglycol groups, $R^{34}$ is an alkyl group with hydrocarbon chain length from 1 to 10 carbon atoms;

or mixtures thereof.

The composition of the present invention can additionally comprise a pH adjuster, where the pH adjuster is selected from:

- An organic acid with the general formula: $R^{35}(-OH)s^2(-COOH)m^1$, where $R^{35}$ is an alkyl group with hydrocarbon chain length from 1 to 12 carbon atoms, $s^2$ takes a value from 1 to 12 and denotes the number of hydroxyl groups arranged in the hydrocarbon radical in any order with respect to each other, $m^1$ takes a value from 1 to 4 and denotes the number of carboxyl groups arranged in the hydrocarbon radical in any order with respect to each other;
- A solution of a hydroxide of an alkaline and/or alkaline earth metal, ammonia, primary or tertiary alkylamine, primary or tertiary alkanolamine, primary or tertiary glucamine, basic amino acid, citric acid disodium salt, citric acid trisodium salt or mixtures thereof;

or mixtures thereof.

The composition of the present invention can additionally comprise a chelating agent, where the chelating agent is selected from:

- A trisodium salt of methylglycinediacetic acid, a tetrasodium salt of glutaminediacetic acid, a trisodium salt of ethylenediamine-(N,N)-disuccinate;
- An organic acid or its salt with an alkaline metal, ammonium, alkylammonium, alkanolammonium, glucoammonium: citric acid, malic acid, tartaric acid, glutaric acid, adipic acid, glucuronic acid, galacturonic acid, galactaric acid, gluconic acid, phytic acid, polytaconic acid, polyacrylic acid, polymethacrylic acid, a copolymer of acrylic and maleic acids, or an organic acid with the general formula $R^{36}(-OH)s^3(-COOH)m^2$, where $R^{36}$ is an alkyl group with hydrocarbon chain length from 1 to 12 carbon atoms, $s^3$ takes a value from 1 to 12 and denotes the number of hydroxyl groups arranged in the hydrocarbon radical in any order with respect to each other, $m^2$ takes a value from 1 to 4 and denotes the number of carboxyl groups arranged in the hydrocarbon radical in any order with respect to each other;

or mixtures thereof.

The composition of the present invention can additionally comprise a soil redeposition inhibitor, where the soil redeposition inhibitor is selected from:

- A polysaccharide derivative selected from: a sodium salt of carboxymethylpolysaccharide, hydroxyalkylpolysaccharide, alkylpolysaccharide or mixtures thereof;
- Polyvynilpyrrolidone;
- A water-soluble salt of polyacrylic acid, or polymethacrylic acid, or a copolymer of akrylic/methacrylic, or maleic acid;

or mixtures thereof.

The composition of the present invention can additionally comprise an anti-foaming agent, where the anti-foaming agent is selected from:

- A higher carboxylic acid with the general formula: $R^{37}-CO_2H$, where $R^{37}$ is an alkyl and/or alkenyl group with hydrocarbon chain length from 5 to 21 carbon atoms;
- A higher carboxylic alcohol with the general formula: $R^{38}-COH$, where $R^{38}$ is an alkyl and/or alkenyl group with hydrocarbon chain length from 5 to 21 carbon atoms;
- A higher carboxylic alcohol ether with the general formula: $R^{39}-O-R^{40}$, where $R^{39}$ and $R^{40}$ independently represent an alkyl and/or alkenyl group with hydrocarbon chain length from 4 to 22 carbon atoms;
- A bisamide of alkyldiamine and a higher carboxylic acid with the general formula: $R^{41}-C(O)-NH-R^{42}-NH-C(O)-R^{43}$, where $R^{41}$ and $R^{43}$ independently represent an alkyl and/or alkenyl group with hydrocarbon chain length from 5 to 21 carbon atoms and $R^{42}$ is an alkyl group with hydrocarbon chain length from 1 to 12 carbon atoms;

or mixtures thereof.

The composition of the present invention can additionally comprise a preservative, where the preservative is selected from:

- An organic acid or its salt with an alkaline or alkaline-earth metal, ammonium, alkylammonium, alkanolammonium, glucoammonium: benzoic acid, sorbic acid, 4-methoxybenzoic acid, salicylic acid, undecylenic acid;
- An organic alcohol or phenol: phenoxyethanol, benzyl alcohol, caprylyl glycol, ethylhexylglycerol, phenethyl alcohol, 3-methyl-4-isopropylphenol, 2,4-dichlorobenzyl alcohol;
- A biocide of a wide range of action: benzisothiazolinone, methylisothiazolinone, dodecyldipropylene triamine;
- A fungicide: sodium pyrithione, climbazole;

or mixtures thereof.

In the second aspect the invention relates to use of the composition of the present invention in a detergent.

The detergent can comprise 0.50-50.25 % wt. of the composition of the present invention.

The detergent can be used for cleavage of low-melting acylglycerides of different spatial configurations, for control of foam stability and/or for hydrophilization of surfaces.

The technical results, among the other things disclosed in the present document and clear to those skilled in the art, related to the biodegradable lipase-based washing composition is increased cleavage of low-melting acylglycerides of different spatial configurations and control of foam stability and hydrophilization of surfaces. The composition is intended to be included in detergents use of which enables control of hydrophilization of surfaces and foam stability upon application of different surfaces and also enables control of kinetics of enzymatic cleavage of low-melting saturated and/or unsaturated acids and their monoacyl-, diacyl- and triacylglycerides with different numbers of bonds with sp$^2$-hybridization having cis- or trans-configuration.

DETAILED DESCRIPTION OF THE INVENTION

The present invention relates to the composition intended for use in a detergent comprising or consisting of:

(A) Biolipase obtained by the biotechnology method from a microorganism, where the given biolipase has biological activity over 40 LU/g, pH 4.0-7.0 at 25°C; and

(B) An aerobically readily biodegradable surfactant or a mixture of surfactants selected from alkylglucoside with the general formula: $R^1$-O-[G]p$^1$, where $R^1$ is an alkyl and/or alkenyl group with hydrocarbon chain length from 4 to 22 carbon atoms, G is a saccharide fragment of 5 or 6 carbon atoms, $p^1$ takes a value from 1 to 4;

(C) An aerobically readily biodegradable surfactant or a mixture of surfactants selected from alkylcarboxylate, and/or alkyl sulfate, and/or alkyl polyethylene glycol sulfate with the general formula $R^2$-(O-CH$_2$-CH$_2$-O)n$^1$(SO$_3$)n$^2$(CO$_2$)n$^3$X$^1$, where $n^1$ takes a value from 0 to 10 and denotes the number of polyethylene groups, $R^2$ is an alkyl and/or alkenyl group with hydrocarbon chain length from 5 to 22 carbon atoms; $n^2$ takes a value from 0 to 1 and denotes the number of sulfate groups; $n^3$ takes a value from 0 to 1 and denotes the number of carboxyl groups; $X^1$ is an alkaline and/or alkaline earth metal, ammonium, alkylammonium, alkanolammonium, glucoammonium, basic aminoacid cation,

wherein the mass ratio of A, B and C components makes up (0.0003-0.25):(1-25):(0.5-25), respectively.

The term "comprising" means that the composition of the present invention can comprise, apart from the enumerated and specified ingredients, other ingredients, too, including specific ingredients intended for use in household chemicals.

The term "consisting of" means that the composition of the present invention does not comprise, apart from the enumerated and specified ingredients, other ingredients and can be used as it is.

The mass content of Component A in the given mass ratio can make up 0.0003, 0.001, 0.01, 0.1, 0.2 or 0.25 or any values between the specified ones.

The mass content of Component B in the given mass ratio can make up 1, 5, 10, 15, 20 or 25 or any values between the specified ones.

The mass content of Component C in the given mass ratio can make up 0,5, 1, 5, 10, 15, 20 or 25 or any values between the specified ones.

The composition of the present invention can additionally comprise an anionic surfactant, wherein the anionic surfactant is selected from:

- A salt of a higher fatty acid amide and methylglycine with the general formula $R^3$-C(O)-N(-CH$_3$)-CH$_2$-CO$_2$X$^2$, where $R^3$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, 22, 23, 24, 25, and $X^2$ is an alkaline and/or alkaline earth metal, ammonium, alkylammonium, alkanolammonium, glucoammonium cation;

- Alkyl polyethyleneglycolcarboxylate with the general formula: $R^4$-O(-CH$_2$-CH$_2$-O-)n$^4$CH$_2$-CO$_2$X$^3$, where n$^4$ takes a value from 1 to 15, for example, 1, 2, 3, 4, 5, 6, 7, 8, 9, 10, 11, 12, 13, 14, 15, and denotes the number of polyethyleneglycol groups, $R^4$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22, and $X^3$ is an alkaline and/or alkaline earth metal, ammonium, alkylammonium, alkanolammonium, glucoammonium cation;
- A disubstituted salt of 2-sulfocarboxylic acid with the general formula: $R^5$-CH(-SO$_3$X$^4$)-CO$_2$X$^4$, where $R^5$ is an alkyl and/or alkenyl group with the hydrocarbon chain length from 4 to 20 carbon atoms, for example, 4, 5, 6, 7, 8, 9, 10, 11, 12, 13, 14, 15, 16, 17, 18, 19, 20, and $X^4$ is an alkaline and/or alkaline earth metal, ammonium, alkylammonium, alkanolammonium, glucoammonium cation;
- A mono- or disubstituted salt of a higher carboxylic acid amide and glutamic acid with the general formula: $R^6$-C(O)-NH-CH(-CH$_2$-CH$_2$-CO$_2$X$^5$)-CO$_2$X$^5$, where $R^6$ is an alkyl and/or alkenyl group with hydrocarbon chain length from 5 to 21 carbon atoms, for example, 5, 6, 7, 8, 9, 10, 11, 12, 13, 14, 15, 16, 17, 18, 19, 20, 21, and $X^5$ is an alkaline and/or alkaline earth metal, ammonium, alkylammonium, alkanolammonium, glucoammonium or hydrogen cation;
- A salt of a higher fatty acid amide and glycine with the general formula: $R^7$-C(O)-NH-CH$_2$-CO$_2$X$^6$, where $R^7$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $X^6$ is an alkaline and/or alkaline earth metal, ammonium, alkylammonium, alkanolammonium, glucoammonium cation;
- A salt of a higher fatty acid amide and alanine with the general formula: $R^8$-C(O)-NH-CH(-CH$_3$)-CO$_2$X$^7$, where $R^8$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $X^7$ is an alkaline and/or alkaline earth metal, ammonium, alkylammonium, alkanolammonium, glucoammonium cation;
- A salt of a higher fatty acid amide and 2-aminomethylethanesulfonic acid with the general formula: $R^9$-C(O)-N(-CH$_3$)-CH$_2$-CH$_2$-SO$_3$X$^8$, where $R^9$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $X^8$ is an alkaline and/or alkaline earth metal, ammonium, alkylammonium, alkanolammonium, glucoammonium cation;
- Alkylpolyglucoside hydroxypropylsulfonate with the general formula: $R^{10}$-O-[G]p$^2$-O-CH$_2$-CH(-OH)-CH$_2$-SO$_3$X$^9$, where $R^{10}$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22, G is a saccharide fragment comprising 5 or 6 carbon atoms, p$^2$ takes a value from 1 to 4, for example, 1, 2, 3, 4, and $X^9$ is an alkaline and/or alkaline earth metal, ammonium, alkylammonium, alkanolammonium, glucoammonium cation;
- Alkylpolyglucoside carboxylate with the general formula: $R^{11}$-O-[G]p$^3$-O-CH$_2$-CO$_2$X$^{10}$, where $R^{11}$ is an alkyl and/or alkenyl group with hydrocarbon chain length from 6 to 22 carbon atoms, G is a saccharide fragment comprising 5 or 6 carbon atoms, p$^3$ takes a value from 1 to 4, for example, 1, 2, 3, 4, and $X^{10}$ is an alkaline and/or alkaline earth metal, ammonium, alkylammonium, alkanolammonium, glucoammonium cation;
- A salt of a higher fatty acid amide and threonine with the general formula: $R^{12}$-C(O)-NH-CH(-CH(-OH)-CH$_3$)-CO$_2$X$^{11}$, where $R^{12}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $X^{11}$ is an alkaline and/or alkaline earth metal, ammonium, alkylammonium, alkanolammonium, glucoammonium cation;
- A salt of a higher fatty acid amide and an aminoacid obtained by hydrolysis of proteins from plant raw materials, with the general formula: $R^{13}$-C(O)-AAX$^{12}$, where $R^{13}$ is an alkyl and/or alkenyl group with hydrocarbon chain length from 5 to 21 carbon atoms, for example, 5, 6, 7, 8, 9, 10, 11, 12, 13, 14, 15, 16, 17, 18, 19, 20, 21, AA is an aminoacid or peptide obtained during hydrolysis of plant protein and $X^{12}$ is an alkaline and/or alkaline earth metal, ammonium, alkylammonium, alkanolammonium, glucoammonium cation;

or mixtures thereof.

The composition of the present invention can additionally comprise an amphoteric surfactant, where the amphoteric surfactant is selected from:

- A disubstituted salt of acylamphodiacetate with the general formula: $R^{14}$-C(O)-NH-CH$_2$-CH$_2$-N(-CH$_2$-CO$_2$X$^{13}$)-CH$_2$-CH$_2$-O-CH$_2$-CO$_2$X$^{13}$, where $R^{14}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $X^{13}$ is an alkaline and/or alkaline earth metal, ammonium, alkylammonium, alkanolammonium, glucoammonium cation;
- An acylamphoacetate salt with the general formula: $R^{15}$-C(O)-NH-CH$_2$-CH$_2$-N(-CH$_2$-CO$_2$X$^{14}$)-CH$_2$-CH$_2$-OH, where $R^{15}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $X^{14}$ is an alkaline and/or alkaline earth metal, ammonium, alkylammonium, alkanolammonium, glucoammonium cation;

- An alkylamphoacetate salt with the general formula: $R^{16}$-C(=N-$CH_2$-$CH_2$-N((-$CH_2$-$CH_2$-OH)-$CH_2$-$CO_2X^{15}$)-), where $R^{16}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $X^{15}$ is an alkaline and/or alkaline earth metal, ammonium, alkylammonium, alkanolammonium, glucoammonium cation;

- Acylamidoalkylbetaine with the general formula: $R^{17}$-C(O)-NH-$R^{18}$-N((-$CH_3$)$_2$)-$CH_2$-$CO_2$ , where $R^{17}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $R^{18}$ is an alkyl group with the hydrocarbon chain length from 1 to 4 carbon atoms, for example, 1, 2, 3, 4;

- Acylamidoalkylhydroxysultaine with the general formula: $R^{19}$-C(O)-NH-$R^{20}$-N(-$CH_3$)$_2$-$CH_2$-CH(-OH)-$CH_2$-$SO_3$, where $R^{19}$ is an alkyl and/or alkenyl group with hydrocarbon chain length from 5 to 21 carbon atoms, for example, 5, 6, 7, 8, 9, 10, 11, 12, 13, 14, 15, 16, 17, 18, 19, 20, 21, and $R^{20}$ is an alkyl group with hydrocarbon chain length from 1 to 4 carbon atoms, for example, 1, 2, 3, 4;

- Acylamidoalkylamine oxide with the general formula: $R^{21}$-C(O)-NH-$R^{22}$-N(-$CH_3$)$_2$-O, where $R^{21}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $R^{22}$ is an alkyl group with the hydrocarbon chain length from 1 to 4 carbon atoms, for example, 1, 2, 3, 4;

- Alkylbetaine with the general formula: $R^{23}$-N((-$CH_3$)$_2$)-$CH_2$-$CO_2$, where $R^{23}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21;

- Alkylhydroxysulfate with the general formula: $R^{24}$-N(-$CH_3$)$_2$-$CH_2$-CH(-OH)-$CH_2$-$SO_3$, where $R^{24}$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22;

- Alkylsulfate with the general formula: $R^{25}$-N(-$CH_3$)$_2$-$CH_2$-$CH_2$-$CH_2$-$SO_3$, where $R^{25}$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22;

- Alkylamine oxide with the general formula: $R^{26}$-N(-$CH_3$)$_2$-O, where $R^{26}$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22;

or mixtures thereof.

The composition of the present invention can additionally comprise a non-ionic surfactant, where the non-ionic surfactant is selected from:

- Alkylpolyethyleneglycol with the general formula: $R^{27}$-O(-$CH_2$-$CH_2$-O-)$n^5$H, where $n^5$ takes a value from 2 to 20, for example, 2, 3, 4, 5, 6, 7, 8, 9, 10, 11, 12, 13, 14, 15, 16, 17, 18, 19, 20, and denotes the number of polyethyleneglycol groups and $R^{27}$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22;

- Alkylpolyethylene/propyleneglycol with the general formula: $R^{28}$-O(-$CH_2$-$CH_2$-O-)$n^6$(-CH(-$CH_3$)-$CH_2$-O-)$n^7$H, where $n^6$ takes a value from 2 to 20, for example, 2, 3, 4, 5, 6, 7, 8, 9, 10, 11, 12, 13, 14, 15, 16, 17, 18, 19, 20, and denotes the number of polyethyleneglycol groups, $n^7$ takes a value from 2 to 20, for example, 2, 3, 4, 5, 6, 7, 8, 9, 10, 11, 12, 13, 14, 15, 16, 17, 18, 19, 20, and denotes the number of polypropyleneglycol groups and $R^{28}$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22;

- Dialkylpolyethyleneglycol with the general formula: $R^{29}$-O(-$CH_2$-$CH_2$-O-)$n^8R^{30}$, where $n^8$ takes a value from 2 to 20, for example, 2, 3, 4, 5, 6, 7, 8, 9, 10, 11, 12, 13, 14, 15, 16, 17, 18, 19, 20, and denotes the number of polyethyleneglycol groups, $R^{29}$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22, and $R^{30}$ is an alkyl and/or alkenyl group with the hydrocarbon chain length from 1 to 12 carbon atoms, for example, 1, 2, 3, 4, 5, 6, 7, 8, 9, 10, 11, 12;

- Dialkylpolyethylene/propyleneglycol with the general formula: $R^{31}$-O(-$CH_2$-$CH_2$-O-)$n^9$(-CH(-$CH_3$)-$CH_2$-O-)$n^{10}$-$R^{32}$, where $n^9$ takes a value from 2 to 20, for example, 2, 3, 4, 5, 6, 7, 8, 9, 10, 11, 12, 13, 14, 15, 16, 17, 18, 19, 20, and denotes the number of polyethyleneglycol groups, $n^{10}$ takes a value from 2 to 20, for example, 2, 3, 4, 5, 6, 7, 8, 9, 10, 11, 12, 13, 14, 15, 16, 17, 18, 19, 20, and denotes the number of polypropyleneglycol groups, $R^{31}$ is an alkyl and/or alkenyl group with the hydrocarbon chain length from 6 to 22 carbon atoms, for example, 6, 7, 8, 9, 10, 11, 12, 13, 14, 15, 16, 17, 18, 19, 20, 21, 22, and $R^{32}$ is an alkyl and/or alkenyl group with the hydrocarbon chain length from 1 to 12 carbon atoms, for example, 1, 2, 3, 4, 5, 6, 7, 8, 9, 10, 11, 12;

or mixtures thereof.

The composition of the present invention can additionally comprise a dispersion medium for a polysaccharide/solvent, where the dispersion medium for a polysaccharide/solvent is selected from:

- An organic alcohol with the general formula: $R^{33}(-OH)s^1$, where $R^{33}$ is an alkyl group with the hydrocarbon chain length from 3 to 12 carbon atoms, for example, 3, 4, 5, 6, 7, 8, 9, 10, 11, 12, $s^1$ takes a value from 1 to 12, for example, 1, 2, 3, 4, 5, 6, 7, 8, 9, 10, 11, 12, and denotes the number of hydroxyl groups arranged in the hydrocarbon radical in any order with respect to each other;
- Alkylpolypropyleneglycol with the general formula: $H(-CH(-CH_3)-CH_2-O-)n^{11}R^{34}$, where $n^{11}$ takes a value from 2 to 10, for exmple, 2, 3, 4, 5, 6, 7, 8, 9, 10, and denotes the number of polypropyleneglycol groups, $R^{34}$ is an alkyl group with the hydrocarbon chain length from 1 to 10 carbon atoms, for example, 1, 2, 3, 4, 5, 6, 7, 8, 9, 10;

or mixtures thereof.

The composition of the present invention can additionally comprise a pH adjuster, where the pH adjuster is selected from:

- An organic alcohol with the general formula: $R^{35}(-OH)s^2(-COOH)m^1$, where $R^{35}$ is an alkyl group with the hydrocarbon chain length from 1 to 12 carbon atoms, for example, 1, 2, 3, 4, 5, 6, 7, 8, 9, 10, 11, 12, $s^2$ takes a value from 1 to 12, for example, 1, 2, 3, 4, 5, 6, 7, 8, 9, 10, 11, 12, and denotes the number of hydroxyl groups arranged in the hydrocarbon radical in any order with respect to each other, $m^1$ takes a value from 1 to 4, for example, 1, 2, 3, 4, and denotes the number of carboxyl groups arranged in the hydrocarbon radical in any order with respect to each other;
- A solution of a hydroxide of an alkaline and/or alkaline earth metal, ammonia, primary or tertiary alkylamine, primary or tertiary alkanolamine, primary or tertiary glucamine, basic amino acid, citric acid disodium salt, citric acid trisodium salt or mixtures thereof;

or mixtures thereof.

The composition of the present invention can additionally comprise a chelating agent, where the chelating agent is selected from:

- A trisodium salt of methylglycinediacetic acid, a tetrasodium salt of glutaminediacetic acid, a trisodium salt of ethylenediamine-(N,N)-disuccinate;
- An organic acid or its salt with an alkaline metal, ammonium, alkylammonium, alkanolammonium, glucoammonium: citric acid, malic acid, tartaric acid, glutaric acid, adipic acid, glucuronic acid, galacturonic acid, galactaric acid, gluconic acid, phytic acid, polytaconic acid, polyacrylic acid, polymethacrylic acid, a copolymer of acrylic and maleic acids, or an organic acid with the general formula $R^{36}(-OH)s^3(-COOH)m^2$, where $R^{36}$ is an alkyl group with hydrocarbon chain length from 1 to 12 carbon atoms, for example, 1, 2, 3, 4, 5, 6, 7, 8, 9, 10, 11, 12, $s^3$ takes a value from 1 to 12, for example, 1, 2, 3, 4, 5, 6, 7, 8, 9, 10, 11, 12, and denotes the number of hydroxyl groups arranged in the hydrocarbon radical in any order with respect to each other, $m^2$ takes a value from 1 to 4 and denotes the number of carboxyl groups arranged in the hydrocarbon radical in any order with respect to each other;

or mixtures thereof.

The composition of the present invention can additionally comprise a soil redeposition inhibitor, where the soil redeposition inhibitor is selected from:

- A polysaccharide derivative selected from: a sodium salt of carboxymethylpolysaccharide, hydroxyalkylpolysaccharide, alkylpolysaccharide or mixtures thereof;
- Polyvynilpyrrolidone;
- A water-soluble salt of polyacrylic acid, or polymethacrylic acid, or a copolymer of akrylic/methacrylic, or maleic acid;

or mixtures thereof.

The composition of the present invention can additionally comprise an anti-foaming agent, where the anti-foaming agent is selected from:

- A higher carboxylic acid with the general formula: $R^{37}-CO_2H$, where $R^{37}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21;
- A higher carboxylic alcohol with the general formula: $R^{38}-COH$, where $R^{38}$ is an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21;

- A higher carboxylic alcohol ether with the general formula: $R^{39}$-O-$R^{40}$, where $R^{39}$ and $R^{40}$ independently represent an alkyl and/or alkenyl group with the hydrocarbon chain length from 4 to 22 carbon atoms, for example, 4, 5, 6, 7, 8, 9, 10, 11, 12, 13, 14, 15, 16, 17, 18, 19, 20, 21, 22;
- A bisamide of alkyldiamine and a higher carboxylic acid with the general formula: $R^{41}$-C(O)-NH-$R^{42}$-NH-C(O)-$R^{43}$, where $R^{41}$ and $R^{43}$ independently represent an alkyl and/or alkenyl group with the hydrocarbon chain length from 5 to 21 carbon atoms, for example, 5, 6, 7, 8, 9, 10, 11, 12, 13, 14, 15, 16, 17, 18, 19, 20, 21, and $R^{42}$ is an alkyl group with the hydrocarbon chain length from 1 to 12 carbon atoms, for example, 1, 2, 3, 4, 5, 6, 7, 8, 9, 10, 11, 12;

or mixtures thereof.

The composition of the present invention can additionally comprise a preservative, where the preservative is selected from:

- An organic acid or its salt with an alkaline or alkaline-earth metal, ammonium, alkylammonium, alkanolammonium, glucoammonium: benzoic acid, sorbic acid, 4-methoxybenzoic acid, salicylic acid, undecylenic acid;
- An organic alcohol or phenol: phenoxyethanol, benzyl alcohol, caprylyl glycol, ethylhexylglycerol, phenethyl alcohol, 3-methyl-4-isopropylphenol, 2,4-dichlorobenzyl alcohol;
- A biocide of a wide range of action: benzisothiazolinone, methylisothiazolinone, dodecyldipropylene triamine;
- A fungicide: sodium pyrithione, climbazole;

or mixtures thereof.

The present invention also relates to use of the composition of the present invention in a detergent.

The detergent can comprise 0.50-50.25 % wt, for example, 0.50, 1, 5, 10, 15, 20, 25, 30, 35, 40, 45, 50, 50.25% wt. of the composition of the present invention.

The detergent can be used for cleavage of low-melting acylglycerides of different spatial configurations, for control of foam stability and/or for hydrophilization of surfaces.

The composition can be different in that biolipase represents lipase or lipase obtained by the biotechnology method from microorganisms. Lipase can represent a substance or commercially available product with the registration number CAS 9001-62-1.

The composition can be different in that glucoside can be a decylglucoside such as Plantacare 2000 UP, Glucopon 215 UP or Natural APG HG 0814CM, a cocoglucoside such as Natural APG HG 0814R37 or Plantacare 818 UP or another commercially available analog. The specified products are commercially available products and are intended for inclusion in formulations.

The composition can differ in that alkyl and/or alkyl sulfate and/or alkyl polyethyleneglycol sulfate can be C12-C18 monoalkyl sulfate, such as Sulfopon G1218 or Sulfopon G1218 MB, C10-16 alkyl polyethyleneglycol sulfate 1-3 EO, such as EMAL 270D, potassium cocoate, such as Mackadet 40K or Eurasol KPZ SG or another commercially available analog. The specified products are commercially available products and are intended for inclusion in formulations.

The detergent of the present invention can comprise 0.50-50.25 % wt. of the composition of the present invention. For example, the detergent of the present invention can comprise 0.50, 1, 5, 10, 15, 20, 25, 30, 35, 40, 45, 50 or 50.25 %wt. or any values between the specified ones of the composition of the present invention.

The present invention also relates to a dishwashing detergent comprising 1.50-50.25 %wt. of the composition of the present invention.

The present invention also relates to a floor, toilet bowl, wash bowl, bathtub or glass cleaner comprising 1.50-50.25 %wt. of the composition of the present invention.

The present invention also relates to an all-purpose surface cleaner comprising 1.50-50.25 %wt. of the composition of the present invention.

The present invention also relates to a drain cleaner comprising 1.50-50.25 %wt. of the composition of the present invention.

The present invention also relates to a laundry detergent, delicate laundry detergent or baby laundry detergent comprising 1.50-50.25 %wt. of the composition of the present invention.

The present invention also relates to a powder laundry detergent comprising 1.50-50.25 %wt. of the composition of the present invention.

The detergent or composition of the present invention can comprise, %wt.:

| | |
|---|---|
| lipase *Lipase* | 0.0003-0.25%, |
| Alkylglucoside with the general formula: $R^1$-O-[G]$p^1$ | 1.00-25.00%, |

(continued)

| Alkyl and/or alkyl sulfate and/or alkyl polyethyleneglycol sulfate with the general formula $R^2$-(O-$CH_2$-$CH_2$-O)$n^1$($SO_3$)$n^2$($CO_2$)$n^3X^1$ | 0.5-25.00%. |

The present invention also relates to use of the composition of the present invention for control of hydrophilization of surfaces and foam stability upon application on different surfaces as well as kinetics of enzymatic cleavage of low-melting saturated and/or unsaturated acids and their monoacyl-, diacyl- and triacylglycerides with different numbers of bonds with $sp^2$-hybridization having cis- or trans-configuration on different surfaces, including metal, polymeric, enameled, glass and wooden ones, for maintaining cleanliness and shine for a long time.

The present invention also relates to a highly effective blend intended for use in household chemicals consisting of: 1) the enzymic complex Medley Brilliant 300L; 2) a non-ionic surfactant selected from Natural APG HG 0814CM, Levenol F-200 or a mixture thereof, and 3) an anionic surfactant selected from Eurasol KPZ SG, EMAL 270 D, Sulfopon 1216 G, MASCID 2012 or a mixture of two or more specified surfactants, where the specified ingredients of the blend are taken with the ratio (0.0003-0.25):(1-25):(0.5-25), respectively. The specified products are commercially available products and are intended for inclusion in formulations of household chemicals. This blend has all advantages of the composition described in the present document and is intended for use in household chemicals such as, but not limited to, a dishwashing detergent, floor, toilet bowl, wash bowl, bathtub or glass cleaner, laundry detergent, delicate laundry detergent, baby laundry detergent, powder laundry detergent.

The mass content of Component (1) in the given mass ratio can make up 0.0003, 0.001, 0.01, 0.1, 0.2 or 0.25 or any values between the specified ones. The mass content of Component (2) in the given mass ratio can make up 1, 5, 10, 15, 20 or 25 or any values between the specified ones. The mass content of Component (3) in the given mass ratio can make up 0,5, 1, 5, 10, 15, 20 or 25 or any values between the specified ones.

The composition preferably does not comprise other active and/or auxiliary substances such as washing active agents and/or acceptable auxiliary substances, but it can also comprise them. Such substances represent or can represent agents conventionally used in the field that are well-known to specialists. Addition of the specified agents to the complex of the present invention does not revoke achievement of the technical results, but it can also improve them.

The framework of the invention also encompasses household chemicals such as a dishwashing detergent, floor, toilet bowl, wash bowl, bathtub or glass cleaner, laundry detergent, delicate laundry detergent, baby laundry detergent, powder laundry detergent.

All fractions, parts, percentage by weight, just like fractions, parts, percentage by volume, in the present disclosure are adjusted with respect to the detergent, agent, composition or product to which they are related in the given context.

The auxiliary acceptable substances in the detergent can be selected from the following categories of components.

Anionic surfactants:

1. - A salt of a higher fatty acid amide and methylglycine with the general formula $R^3$-C(O)-N(-$CH_3$)-$CH_2$-$CO_2X^2$, where $R^3$ is an alkyl and/or alkenyl group with hydrocarbon chain length from 5 to 21 carbon atoms and $X^2$ is an alkaline and/or alkaline earth metal, ammonium, alkylammonium, alkanolammonium, glucoammonium cation;

2. - Alkyl polyethylene glycol carboxylate with the general formula: $R^4$-O(-$CH_2$-$CH_2$-O-)$n^4CH_2$-$CO_2X^3$, where $n^4$ takes a value from 1 to 15 and denotes the number of polyethylene glycol groups, $R^4$ is an alkyl and/or alkenyl group with hydrocarbon chain length from 6 to 22 carbon atoms and $X^3$ is an alkaline and/or alkaline earth metal, ammonium, alkylammonium, alkanolammonium, glucoammonium cation;

3. A disubstituted salt of 2-sulfocarboxylic acid with the general formula: $R^5$-CH(-$SO_3X^4$)-$CO_2X^4$, where $R^5$ is an alkyl and/or alkenyl group with hydrocarbon chain length from 4 to 20 carbon atoms and $X^4$ is an alkaline and/or alkaline earth metal, ammonium, alkylammonium, alkanolammonium, glucoammonium cation;

4. A mono- or disubstituted salt of a higher carboxylic acid amide and glutamic acid with the general formula: $R^6$-C(O)-NH-CH(-$CH_2$-$CH_2$-$CO_2X^5$)-$CO_2X^5$, where $R^6$ is an alkyl and/or alkenyl group with hydrocarbon chain length from 5 to 21 carbon atoms and $X^5$ is an alkaline and/or alkaline earth metal, ammonium, alkylammonium, alkanolammonium, glucoammonium or hydrogen cation;

5. A salt of a higher fatty acid amide and glycine with the general formula: $R^7$-C(O)-NH-$CH_2$-$CO_2X^6$, where $R^7$ is an alkyl and/or alkenyl group with hydrocarbon chain length from 5 to 21 carbon atoms and $X^6$ is an alkaline and/or alkaline earth metal, ammonium, alkylammonium, alkanolammonium, glucoammonium cation;

6. A salt of a higher fatty acid amide and alanine with the general formula: $R^8$-C(O)-NH-CH(-CH$_3$)-CO$_2$X$^7$, where $R^8$ is an alkyl and/or alkenyl group with hydrocarbon chain length from 5 to 21 carbon atoms and X' is an alkaline and/or alkaline earth metal, ammonium, alkylammonium, alkanolammonium, glucoammonium cation;

7. A salt of a higher fatty acid amide and 2-aminomethylethanesulfonic acid with the general formula: $R^9$-C(O)-N(-CH$_3$)-CH$_2$-CH$_2$-SO$_3$X$^8$, where $R^9$ is an alkyl and/or alkenyl group with hydrocarbon chain length from 5 to 21 carbon atoms, and X$^8$ is an alkaline and/or alkaline earth metal, ammonium, alkylammonium, alkanolammonium, glucoammonium cation;

8. Alkylpolyglucoside hydroxypropylsulfonate with the general formula: $R^{10}$-O-[G]p$^2$-O-CH$_2$-CH(-OH)-CH$_2$-SO$_3$X$^9$, where $R^{10}$ is an alkyl and/or alkenyl group with hydrocarbon chain length from 6 to 22 carbon atoms, G is a saccharide fragment comprising 5 or 6 carbon atoms, p$^2$ takes a value from 1 to 4 and X$^9$ is an alkaline and/or alkaline earth metal, ammonium, alkylammonium, alkanolammonium, glucoammonium cation;

9. Alkylpolyglucoside carboxylate with the general formula: $R^{11}$-O-[G]p$^3$-O-CH$_2$-CO$_2$X$^{10}$, where $R^{11}$ is an alkyl and/or alkenyl group with hydrocarbon chain length from 6 to 22 carbon atoms, G is a saccharide fragment comprising 5 or 6 carbon atoms, p$^3$ takes a value from 1 to 4 and X$^{10}$ is an alkaline and/or alkaline earth metal, ammonium, alkylammonium, alkanolammonium, glucoammonium cation;

10. A salt of a higher fatty acid amide and threonine with the general formula: $R^{12}$-C(O)-NH-CH(-CH(-OH)-CH$_3$)-CO$_2$X$^{11}$, where $R^{12}$ is an alkyl and/or alkenyl group with hydrocarbon chain length from 5 to 21 carbon atoms and X$^{11}$ is an alkaline and/or alkaline earth metal, ammonium, alkylammonium, alkanolammonium, glucoammonium cation;

11. A salt of a higher fatty acid amide and an aminoacid obtained by hydrolysis of proteins from plant raw materials, with the general formula: $R^{13}$-C(O)-AAX$^{12}$, where $R^{13}$ is an alkyl and/or alkenyl group with hydrocarbon chain length from 5 to 21 carbon atoms, AA is an aminoacid or peptide obtained during hydrolysis of plant protein and X$^{12}$ is an alkaline and/or alkaline earth metal, ammonium, alkylammonium, alkanolammonium, glucoammonium cation;

Amphoteric surfactants:

1. A disubstituted salt of acylamphodiacetate with the general formula: $R^{14}$-C(O)-NH-CH$_2$-CH$_2$-N(-CH$_2$-CO$_2$X$^{13}$)-CH$_2$-CH$_2$-O-CH$_2$-CO$_2$X$^{13}$, where $R^{14}$ is an alkyl and/or alkenyl group with hydrocarbon chain length from 5 to 21 carbon atoms and X$^{13}$ is an alkaline and/or alkaline earth metal, ammonium, alkylammonium, alkanolammonium, glucoammonium cation;

2. An acylamphoacetate salt with the general formula: $R^{15}$-C(O)-NH-CH$_2$-CH$_2$-N(-CH$_2$-CO$_2$X$^{14}$)-CH$_2$-CH$_2$-OH, where $R^{15}$ is an alkyl and/or alkenyl group with hydrocarbon chain length from 5 to 21 carbon atoms and X$^{14}$ is an alkaline and/or alkaline earth metal, ammonium, alkylammonium, alkanolammonium, glucoammonium cation;

3. An alkylamphoacetate salt with the general formula: $R^{16}$-C(=N-CH$_2$-CH$_2$-N((-CH$_2$-CH$_2$-OH)-CH$_2$-CO$_2$X$^{15}$)-), where $R^{16}$ is an alkyl and/or alkenyl group with hydrocarbon chain length from 5 to 21 carbon atoms and X$^{15}$ is an alkaline and/or alkaline earth metal, ammonium, alkylammonium, alkanolammonium, glucoammonium cation;

4. Acylamidoalkylbetaine with the general formula: $R^{17}$-C(O)-NH-R$^{18}$-N((-CH$_3$)$_2$)-CH$_2$-CO$_2$, where $R^{17}$ is an alkyl and/or alkenyl group with hydrocarbon chain length from 5 to 21 carbon atoms and $R^{18}$ is an alkyl group with hydrocarbon chain length from 1 to 4 carbon atoms;

5. Acylamidoalkylhydroxysultaine with the general formula: $R^{19}$-C(O)-NH-R$^{20}$-N(-CH$_3$)$_2$-CH$_2$-CH(-OH)-CH$_2$-SO$_3$, where $R^{19}$ is an alkyl and/or alkenyl group with hydrocarbon chain length from 5 to 21 carbon atoms and $R^{20}$ is an alkyl group with hydrocarbon chain length from 1 to 4 carbon atoms;

6. Acylamidoalkylamine oxide with the general formula: $R^{21}$-C(O)-NH-R$^{22}$-N(-CH$_3$)$_2$-O, where $R^{21}$ is an alkyl and/or alkenyl group with hydrocarbon chain length from 5 to 21 carbon atoms and $R^{22}$ is an alkyl group with hydrocarbon chain length from 1 to 4 carbon atoms;

7. Alkylbetaine with the general formula: $R^{23}$-N((-CH$_3$)$_2$)-CH$_2$-CO$_2$, where $R^{23}$ is an alkyl and/or alkenyl group with hydrocarbon chain length from 5 to 21 carbon atoms;

8. Alkylhydroxysultaine with the general formula: $R^{24}$-N(-CH$_3$)$_2$-CH$_2$-CH(-OH)-CH$_2$-SO$_3$, where $R^{24}$ is an alkyl and/or alkenyl group with hydrocarbon chain length from 6 to 22 carbon atoms;

9. Alkylsultaine with the general formula: $R^{25}$-N(-CH$_3$)$_2$-CH$_2$-CH$_2$-CH$_2$-SO$_3$, where $R^{25}$ is an alkyl and/or alkenyl group with hydrocarbon chain length from 6 to 22 carbon atoms;

10. Alkylamine oxide with the general formula: $R^{26}$-N(-CH$_3$)$_2$-O, where $R^{26}$ is an alkyl and/or alkenyl group with hydrocarbon chain length from 6 to 22 carbon atoms;

Non-ionic surfactants:

1. Alkylpolyethyleneglycol with the general formula: $R^{27}$-O(-$CH_2$-$CH_2$-O-)$n^5$H, where $n^5$ takes a value from 2 to 20 and denotes the number of polyethyleneglycol groups, $R^{27}$ is an alkyl and/or alkenyl group with hydrocarbon chain length from 6 to 22 carbon atoms;

2. Alkylpolyethylene/propyleneglycol with the general formula: $R^{28}$-O(-$CH_2$-$CH_2$-O-)$n^6$(-CH(-$CH_3$)-$CH_2$-O-)$n^7$H, where $n^6$ takes a value from 2 to 20 and denotes the number of polyethyleneglycol groups, $n^7$ takes a value from 2 to 20 and denotes the number of polypropyleneglycol groups, $R^{28}$ is an alkyl and/or alkenyl group with hydrocarbon chain length from 6 to 22 carbon atoms;

3. Dialkylpolyethyleneglycol with the general formula: $R^{29}$-O(-$CH_2$-$CH_2$-O-)$n^8R^{30}$, where $n^8$ takes a value from 2 to 20 and denotes the number of polyethyleneglycol groups, $R^{29}$ is an alkyl and/or alkenyl group with hydrocarbon chain length from 6 to 22 carbon atoms, $R^{30}$ is an alkyl and/or alkenyl group with hydrocarbon chain length from 1 to 12 carbon atoms;

4. Dialkylpolyethylene/propyleneglycol with the general formula: $R^{31}$-O(-$CH_2$-$CH_2$-O-)$n^9$(-CH(-$CH_3$)-$CH_2$-O-)$n^{10}$-$R^{32}$, where $n^9$ takes a value from 2 to 20 and denotes the number of polyethyleneglycol groups, $n^{10}$ takes a value from 2 to 20 and denotes the number of polypropyleneglycol groups, $R^{31}$ is an alkyl and/or alkenyl group with hydrocarbon chain length from 6 to 22 carbon atoms, $R^{32}$ is an alkyl and/or alkenyl group with hydrocarbon chain length from 1 to 12 carbon atoms.

A dispersion medium for a polysaccharide/solvent:

1. An organic alcohol with the general formula: $R^{33}$(-OH)$s^1$, where $R^{33}$ is an alkyl group with hydrocarbon chain length from 3 to 12 carbon atoms, $s^1$ takes a value from 1 to 12 and denotes the number of hydroxyl groups arranged in the hydrocarbon radical in any order with respect to each other;

2. Alkylpolypropyleneglycol with the general formula: H(-CH(-$CH_3$)-$CH_2$-O-)$n^{11}R^{34}$, where $n^{11}$ takes a value from 2 to 10 and denotes the number of polypropyleneglycol groups, $R^{34}$ is an alkyl group with hydrocarbon chain length from 1 to 10 carbon atoms.

PH adjusters:

1. Organic acids with the general formula: $R^{35}$(-OH)$s^2$(-COOH)$m^1$, where $R^{35}$ is an alkyl group with hydrocarbon chain length from 1 to 12 carbon atoms, $s^2$ takes a value from 1 to 12 and denotes the number of hydroxyl groups arranged in the hydrocarbon radical in any order with respect to each other, $m^1$ takes a value from 1 to 4 and denotes the number of carboxyl groups arranged in the hydrocarbon radical in any order with respect to each other;

2. Solutions of hydroxides of alkaline and/or alkaline earth metals, ammonia, primary or tertiary alkylamines, primary or tertiary alkanolamines, primary or tertiary glucamines, a basic amino acid, a citric acid disodium salt, a citric acid trisodium salt.

A chelating agent:

1. A trisodium salt of methylglycinediacetic acid, a tetrasodium salt of gluteminediacetic acid, a trisodium salt of ethylenediamine-(N,N)-disuccinate;

2. Organic acids as well as salts of alkaline metals, ammonium, alkylammonium, alkanolammonium, glucoammonium corresponding to the following acids: citric acid, malic acid, tartaric acid, glutaric acid, adipic acid, glucuronic acid, galacturonic acid, galactaric acid, gluconic acid, phytic acid, polytaconic acid, polyacrylic acid, polymethacrylic acid, a copolymer of acrylic and maleic acids, as well as organic acids with the general formula $R^{36}$(-OH)$s^3$(-COOH)$m^2$, where $R^{36}$ is an alkyl group with hydrocarbon chain length from 1 to 12 carbon atoms, $s^3$ takes a value from 1 to 12 and denotes the number of hydroxyl groups arranged in the hydrocarbon radical in any order with respect to each other, $m^2$ takes a value from 1 to 4 and denotes the number of carboxyl groups arranged in the hydrocarbon radical in any order with respect to each other.

Soil redeposition inhibitors:

1. Derivatives of polysaccharides: carboxymethylpolysaccharide sodium salt, hydroxyalkylpolysaccharide, alkylpolysaccharide;
2. Polyvinylpyrrolidone;
3. Water-soluble salts of polyacrylic acid, polymethacrylic acid, a copolymer of akrylic/methacrylic and maleic acid.

Anti-foaming agents:

1. Higher carboxylic acids with the general formula: $R^{37}-CO_2H$, where $R^{37}$ is an alkyl and/or alkenyl group with hydrocarbon chain length from 5 to 21 carbon atoms;
2. Higher carboxylic alcohols with the general formula: $R^{38}-COH$, where $R^{38}$ is an alkyl and/or alkenyl group with hydrocarbon chain length from 5 to 21 carbon atoms;
3. Ethers of higher carboxylic alcohols with the general formula: $R^{39}-O-R^{40}$, where $R^{39}$, $R^{40}$ are an alkyl and/or alkenyl group with hydrocarbon chain length from 4 to 22 carbon atoms;
4. Bisamides of alkyldiamines and higher carboxylic acids with the general formula: $R^{41}-C(O)-NH-R^{42}-NH-C(O)-R^{43}$, where $R^{41}$, $R^{43}$ are an alkyl and/or alkenyl group with hydrocarbon chain length from 5 to 21 carbon atoms and $R^{42}$ is an alkyl group with hydrocarbon chain length from 1 to 12 carbon atoms;

Preservatives:

1. Organic acids and salts of alkaline and alkaline-earth metals, ammonium, alkylammonium, alkanolammonium, glucoammonium corresponding to the following acids: benzoic acid, sorbic acid, 4-methoxybenzoic acid, salicylic acid, undecylenic acid;
2. Organic alcohols and phenols: phenoxyethanol, benzyl alcohol, caprylyl glycol, ethylhexylglycerol, phenethyl alcohol, 3-methyl-4-isopropylphenol, 2,4-dichlorobenzyl alcohol;
3. Biocides of a wide range of action: benzisothiazolinone, methylisothiazolinone, dodecyldipropylene triamine;
4. Fungicides: sodium pyrithione, climbazole.

EXPERIMENTAL DATA

The examples included in the present description do not limit the claimed invention and are given only with the aim of illustration and confirmation of achievement of expected technical results. These examples are among many experimental data obtained by the authors of the invention that prove effectiveness of the detergents within the scope of the invention.

**Example 1.**

The components to be included in the composition of the present invention were studied in the formulation of different detergents. A liquid detergent (formula No. 1), specifically, an all-purpose cleaner for all surfaces, was prepared within the scope of the present invention (Table 1).

Table 1. Formulation of the all-purpose cleaner with the claimed composition

| No. | Component | Content, %wt. |
|---|---|---|
| 1 | Purified water | up to 100 |

(continued)

| No. | Component | Content, %wt. |
|---|---|---|
| 2 | Alkylcarboxylate and/or alkyl sulfate and/or alkyl polyethylene glycol sulfate with the general formula $R^2$-(O-$CH_2$-$CH_2$-O)$n^1$($SO_3$)$n^2$($CO_2$)$n^3X^1$, where $n^1$ takes a value from 0 to 10 and denotes the number of polyethylene groups, $R^2$ is an alkyl and/or alkenyl group with hydrocarbon chain length from 5 to 22 carbon atoms; $n^2$ takes a value from 0 to 1 and denotes the number of sulfate groups; $n^3$ takes a value from 0 to 1 and denotes the number of carboxyl groups; $X^1$ represents an alkaline and/or alkaline earth metal, ammonium, alkylammonium, alkanolammonium, glucoammonium, basic amino acid cation, specifically, C10-16 alkyl carboxylate | 1-15 |
| 3 | Alkylglucoside with the general formula: $R^1$-O-[G]$p^1$, where $R^1$ is an alkyl and/or alkenyl group with hydrocarbon chain length from 4 to 22 carbon atoms, G is a saccharide fragment of 5 or 6 carbon atoms, $p^1$ can take a value from 1 to 4 | 2-15 |
| 4 | Biolipase | 0.005-0.01 |
| 5 | Complexing agent | 0.1-0.8 |
| 6 | Cotton extract | 0.05-0.15 |
| 7 | Aqueous solution of citric acid and silver citrate | 0.001-0.1 |
| 8 | Citric acid monohydrate | 0.01-0.3 |
| 9 | Preservative | 0.2-0.8 |

The prepared all-purpose cleaner ensures high effectiveness of cleavage of low-melting saturated and/or unsaturated acids and their monoacyl-, diacyl- and triacylglycerides with different numbers of bonds with $sp^2$-hybridization having cis- or trans-configuration on different surfaces, including metal, polymeric, enameled, glass and wooden ones. It is not corrosive for cleaned surfaces, specifically, it does not leave lime scale, it is stable during storage within 24 months (observation time).

**Example 2.**

The components to be included in the composition of the present invention were studied in the formulation of different detergents. A liquid detergent (formula No. 2), specifically, a liquid dishwashing detergent, was prepared within the scope of the invention (Table 2).

Table 2. Formulation of the liquid dishwashing detergent with the claimed composition

| No. | Component | Content, % wt. |
|---|---|---|
| 1 | Purified water | up to 100 |
| 2 | Alkylcarboxylate and/or alkyl sulfate and/or alkyl polyethylene glycol sulfate with the general formula $R^2$-(O-$CH_2$-$CH_2$-O)$n^1$($SO_3$)$n^2$($CO_2$)$n^3X^1$, where $n^1$ takes a value from 0 to 10 and denotes the number of polyethylene groups, $R^2$ is an alkyl and/or alkenyl group with hydrocarbon chain length from 5 to 22 carbon atoms; $n^2$ takes a value from 0 to 1 and denotes the number of sulfate groups; $n^3$ takes a value from 0 to 1 and denotes the number of carboxyl groups; $X^1$ represents an alkaline and/or alkaline earth metal, ammonium, alkylammonium, alkanolammonium, glucoammonium, basic aminoacid cation, specifically, C10-16 alkyl polyethylene glycol sulfate 1-3 EO | 5-25 |
| 3 | Alkylglucoside with the general formula: $R^1$-O-[G]$p^1$, where $R^1$ is an alkyl and/or alkenyl group with hydrocarbon chain length from 4 to 22 carbon atoms, G is a saccharide fragment of 5 or 6 carbon atoms, $p^1$ can take a value from 1 to 4, specifically, C10-16 alkylpolyglucoside and C8-10 alkylpolyglucoside | 1-17 |

(continued)

| No. | Component | Content, % wt. |
|---|---|---|
| 4 | Biolipase | 0.01-0.25 |
| 5 | Complexing agent | 0.1-1.1 |
| 6 | Cotton extract | 0.02-0.18 |
| 7 | Aqueous solution of citric acid and silver citrate | 0.005-0.2 |
| 8 | Citric acid monohydrate | 0.05-0.8 |
| 9 | Preservative | 0.004-0.1 |
| 10 | Auxiliary substances, if required | 2.0-12.0 |

The prepared liquid dishwashing detergent ensures high effectiveness of cleavage of low-melting saturated and/or unsaturated acids and their monoacyl-, diacyl- and triacylglycerides with different numbers of bonds with $sp^2$-hybridization having cis- or trans-configuration from different surfaces, with any hardness of tap water and at any water temperature. It does not cause changes in gloss or the previous appearance of the surface, leaves no lime scale and scratches, washes off tableware almost completely, suits for washing baby accessories, vegetables and fruits, is stable during storage within 24 months (observation period).

**Example 3.**

The components to be included in the composition of the present invention were studied in the formulation of different detergents. A liquid detergent (formula No.3), specifically, a liquid laundry detergent and stain remover, was prepared within the scope of the invention (Table 3).

Table 3. Formulation of the liquid laundry detergent with the claimed composition

| No. | Component | Content, % wt. |
|---|---|---|
| 1 | Purified water | up to 100 |
| 2 | Alkyl carboxylate and/or alkyl sulfate and/or alkyl polyethylene glycol sulfate with the general formula $R^2$-(O-CH$_2$-CH$_2$-O)n$^1$(SO$_3$)n$^2$(CO$_2$)n$^3$X$^1$, where n$^1$ takes a value from 0 to 10 and denotes the number of polyethylene groups, $R^2$ is an alkyl and/or alkenyl group with hydrocarbon chain length from 5 to 22 carbon atoms; n$^2$ takes a value from 0 to 1 and denotes the number of sulfate groups; n$^3$ takes a value from 0 to 1 and denotes the number of carboxyl groups; X$^1$ represents an alkaline and/or alkaline earth metal, ammonium, alkylammonium, alkanolammonium, glucoammonium, basic aminoacid cation, specifically, C10-16 alkyl sulfate | 1-15 and/or 1-15 |
| 3 | Alkylglucoside with the general formula: $R^1$-O-[G]p$^1$, where $R^1$ is an alkyl and/or alkenyl group with hydrocarbon chain length from 4 to 22 carbon atoms, G is a saccharide fragment of 5 or 6 carbon atoms, p$^1$ can take a value from 1 to 4, specifically, C10-16 alkylpolyglucoside and C8-10 alkylpolyglucoside | 2.5-25 |
| 4 | Biolipase | 0.0002-0.10 |
| 5 | Complexing agent | 0.1-1.1 |
| 6 | Cotton proteins | 0.02-0.18 |
| 7 | Aqueous solution of citric acid and silver citrate | 0.001-0.1 |
| 8 | Citric acid monohydrate | 0.05-0.75 |
| 9 | Preservative | 0.25-0.75 |
| 10 | Auxiliary substances, if required | 2.0-25.0 |

The prepared liquid laundry detergent and stain remover ensures high effectiveness of cleavage of low-melting saturated and/or unsaturated acids and their monoacyl-, diacyl- and triacylglycerides with different numbers of bonds with $sp^2$-hybridization having cis- or trans-configuration from different fabrics, specifically, cotton, synthetic, membrane, linen, woolen, cashmere ones, with any hardness of tap water and at any wash temperature from +20°C to +60°C. It does not change fabric color and does not cause colorant wash-off, it keeps the former look of the product, leaves no film, washes off the fabric surface completely, suits for washing laundry of babies and people with sensitive hand skin, it remains stable during storage within 24 months (observation period).

**Example 4.**

The components of the composition and ready formulations of the detergents specified in Table 4 were checked for conformity to main ecocertificates of Level I - EU Ecolabel, Nordic Swan Ecolabel, Vitality Leaf, as well as requirements of Halal certification.

Table 4. Formulations

| No. | Name of formulation | Components |
|---|---|---|
| 1 | Surface cleaner with the composition | Aqua, Decyl Glucoside, Fatty acids, coco (coconut oil), potassium salts, Tetrasodium Glutamate Diacetate, Glycerin, Sorbitol, Lipase, Citric Acid, Gossypium Herbaceum Extract, Silver Citrate, Preservatives, Any functional additives. |
| 2 | Dishwashing detergent with the composition | Aqua, Poly(oxy-1,2-ethanediyl), .alpha.-sulfo-.omega.- hydroxy-, C10-16-alkyl ethers, sodium salts, Decyl Glucoside, C8-18/18-unsaturated amidopropyl betaine, Glycerin, Sorbitol, Lipase, Citric Acid, Tetrasodium Glutamate Diacetate, Gossypium Herbaceum Extract, Silver Citrate, Preservatives, Any functional additives. |
| 3 | Laundry detergent and stain remover with the composition | Aqua, Glycerin, Decyl Glucoside, Glycereth-6 Laurate, Fatty acids, coco (coconut oil), potassium salt, C8-18/18-unsaturated amidopropyl betaine, Non-ionic ethoxylated fatty alcohol and fatty acid, Trisodium salt of methylglycinediacetic acid, C12-18 alkyl sulfate, Hydrolyzed Cotton Protein, Lipase and other enzymes, Parfum, Sodium Hydroxide, Citric Acid, Preservatives, Any functional additives. |
| 4 | Alternative surface cleaner | Aqua, Propylene Glycol Butyl Ether, Alcohol, Aminomethyl Propanol Cocamidopropyl Hydroxysultaine, Alkyl Dimethyl Ethylbenzyl Ammonium Chloride, Benzalkonium Chloride, Parfum, Propylene Glycol, Tetrasodium EDTA. https://www.info-pg.com/deu/variants/Deutschland?prodForm=799 |
| 5 | Alternative dishwashing detergent | Aqua, Sodium Laureth Sulfate, Lauramine Oxide, Alcohol, Sodium Chloride, Poly[oxy(methyl-1,2-ethanediyl)], alpha.-hydro-.omega.-hydroxy- (n=17), Parfum, PEI/PEG/PPG Copolymer, Phenoxyethanol, Sodium Hydroxide, 4-Methylcyclohexane-1,3-diamine, Limonene, Magnesium Sulfate, Dipropylene Glycol, 2-Methylcyclohexane-1,3-diamine, Citronellol, Benzisothiazolinone, Colorant, Methylisothiazolinone. https://www.info-pg.com/eng/variants/United%20Kingdom?prodForm=507 |
| 6 | Alternative laundry detergent and stain remover | Aqua, MEA-C10-13 Alkyl Benzenesulfonate, MEA-Laureth Sulfate, MEA-citrate, Propylene Glycol, Palm Kernel Fatty Acid, Parfum, Co-polymer of PEG / Vinyl Acetate, C12-14 Pareth-n, Trimonoethanol Etidronate, Hydrogenated Castor Oil, MEA-Palm Kernelate, C15 Pareth-n Sulfated Ethoxylated Hexamethylenediamine Quaternized, PEG/PPG-10/2 Propylheptyl Ether, Glycerin, Ethanolamine, Disodium Distyrylbiphenyl Disulfonate, Citronellol, Sorbitol, Tripropylene Glycol, Protease, Sodium Formate, Alpha-Isomethyl Ionone, Calcium Chloride, Limonene, Phosphodiesterase, Dipropylene Glycol, Amylase, Sodium Chloride, Sodium Acetate, Colorant, Lyase, Cellulase, Mannanase, Benzisothiazolinone, Sodium Hydroxide, Dipropylene Glycol, Disodium Phosphate. https://www.info-pg.com/eng/variants/United%20Kingdom?prodForm=226 |

This ecological marking is recognized in the European Union and in the whole world and is a mark of ecological safety awarded to products and services meeting strict ecological standards. Specifically, EU Ecolabel, Nordic Swan and Vitality Leaf labels support closed-loop economy, encouraging producers to produce less waste and carbon dioxide

$CO_2$ emissions. They are directed at reduction of negative impact on the environment, health, climate and natural resources. Detergents conforming to these ecological certificates consist of ingredients registered in the Detergents Ingredients Database (DID list) or ingredients having an acceptable ability of biodegradation and an acceptable level of toxic substances hazardous for the aquatic environment. In addition, these labels confirm product effectiveness with respect to stubborn stains while meeting high ecological regulations. Below there is assessment of conformity of formulations 1-3 to these ecocertificates and requirements of Halal in relation to controls (Table 5).

Table 5. Conformity of formulations to ecomarking requirements

| Formulation | EU Ecolabel | Nordic Swan | Vitality Leaf | Halal |
|---|---|---|---|---|
| Surface cleaner from Table 1 | conformity | conformity | conformity | conformity |
| Dishwashing detergent from Table 2 | conformity | conformity | conformity | conformity |
| Laundry detergent from Table 3 | conformity | conformity | conformity | conformity |
| Alternative dishwashing detergent No. 4 | non-conformity Parfum, Poly[oxy(methyl-1,2-ethanediyl)]. .alpha.-hydro-.omega.-hydroxy- (n=17) | non-conformity Parfum, Poly[oxy(methyl-1,2-ethanediyl)]. .alpha.-hydro-.omega.-hydroxy- (n=17), Methylisothiazolinone | non-conformity Parfum, Poly[oxy(methyl-1,2-ethanediyl)]. .alpha.-hydro-.omega.hydroxy- (n=17) | non-conformity Alcohol |
| Alternative surface cleaner No. 5 | non-conformity Parfum, Benzalkonium Chloride, Tetrasodium EDTA | non-conformity Parfum, Benzalkonium Chloride, Tetrasodium EDTA | non-conformity Parfum, Benzalkonium Chloride, Tetrasodium EDTA | non-conformity Alcohol |
| Alternative laundry detergent No. 6: | Non-conformity Disodium Phosphate, Parfum | non-conformity Disodium Phosphate, Parfum | Non-conformity Disodium Phosphate, Parfum | conformity |

According to the results of assessment it was revealed that the composition and formulations 1-3 conform to strict requirements of ecological compatibility and environmental safety, specifically, acceptable ability of biodegradation and level of toxic substances hazardous for the aquatic environment. The composition and formulations No. 1-3 do not comprise components restricted by the world certificates of ecological compatibility of products, which conforms to the informed approach to environmental protection and modern consumer preferences concerning natural ecological household chemicals.

Pre-clinical studies were conducted to assess effectiveness and stability of the composition of the present invention.

**Example 5.**

There was a laboratory study of biodegradability of active components in formulations of household chemicals in accordance with the regulating standard OECD 301 B. A solution of the detergent with the concentration from 10 to 20 mg/l was added to a chemically pure culture medium free of other carbon-containing compounds. Then an inoculum of microorganisms was added. In the process of their activity the microorganisms enable biodegradation of organic compounds in an aerobic atmosphere free of carbon dioxide. The released carbon dioxide $CO_2$ is entrapped by supplied oxygen at a rate of 50-100 ml/min, is adsorbed by barium hydroxide $Ba(OH)_2$, then the quantity of formed insoluble barium carbonate $BaCO_3$ is calculated by back titration with a solution of hydrochloric acid with the phenolphthalein indicator. Sodium benzoate with the concentration of 10-20 mg/l with known biodegradability kinetics is used as a control. The experiment is carried out during 28 days at the temperature of $22\pm2°C$, in a dry and light-proof place. The method is accurate and express for assessment of practical biodegradability of carbon-containing compounds, specifically, surfactants, enzymes, functional additives, complexing agents. A washing or cleaning agent is recognized to be biodegradable if during 28 days more than 60% of carbon-containing compounds are converted to carbon dioxide.

Results. Based on the results of testing of the all-purpose cleaner for all surfaces (formulation No.1), liquid dishwashing detergent (formulation No.2), liquid laundry detergent (formulation No.3) it was established that the compositions under study have high biodegradability in accordance with the international standard OECD 301B. The study of kinetics demonstrated high % of composition biodegradability, quick achievement of the indicator $BD_{50}$ - the half-life period showing the time (days) required for degradation of 50% of the formulation to carbon dioxide. The lower the given indicator is, the quicker and easier the given formulation degrades in natural conditions. According to the indicator dynamics in relation to the control sample, % of biodegradability of all formulations 1-3 made up over 70%, while the half-life period made up under 7 days, which speaks of minimum accumulation of components of the composition in the environment and in aqueous organisms. The control sample demonstrated achievement of biodegradability from 90 to 92% and serves as the upper border of the possible result (Table 6).

Table 6. Assessment of biodegradability of samples of detergents with the claimed composition

| Test sample | Naturality $I_{NO}$ ISO 16128 | Biodegradability during 28 days, % | Indicator $BD_{50}$, days |
|---|---|---|---|
| No.1 | 99% | 71% | 7 |
| No.2 | 96% | 79% | 7 |
| No.3 | 95% | 74% | 7 |
| No.4 | <90% | <60% | >28 days |
| No.5 | <70% | >28 days | >28 days |
| No.6 | <70% | >28 days | >28 days |
| Control | 0% | 88% | 4 |

It is difficult to remove complex lipid soils based on low-melting saturated and/or unsaturated acids and their monoacyl-, diacyl- and triacylglycerides with different numbers of bonds with $sp^2$-hybridization having cis- or trans-configuration from different surfaces owing to fixation on surfaces, especially hydrophobic ones, insufficient action of surfactants from household chemicals, despite solubilizing properties of micellae. Often producers use surfactants of synthetic origin from petrochemical sources, incl. non-biodegradable ones, which negatively affects the environment in connection with annual consumption of household chemicals > 11.9 kg per person.

The composition of surfactants of a surface cleaner will mildly remove soils from hydrophobic and hydrophilic surfaces (metal, polymeric, glass, glass-ceramic, ceramic, wooden and other ones) and increase access of biolipase from the claimed composition to hard-to-get invisible areas, thus improving effectiveness of the composition with respect to complex lipid soils, incl. old ones. The composition consists of more than 95% of ingredients of natural and mineral origin, enables replacing surfactants of petrochemical production with surfactants of natural origin completely without reduction of effectiveness of the chemical, which makes it possible to reduce the environmental load, maintain the approach to human health and ensure a high percentage of natural ingredients in the formulation of household chemicals for consumers. The proven aerobic biodegradability of the composition in the formulations of different detergents, specifically, dishwashing detergents, all-purpose surface cleaners and laundry detergents, proves safety for the environment and absence of accumulation in aqueous organisms, aqueous sources and soil after use, maintaining the trend for ecological sustainability.

Thus, combination of the composition components enables achieving a high content of natural ingredients and a high degree of biodegradability as early as 7 days while preserving effectiveness, which enables regular use in formulations of household chemicals for solving of different tasks and conformity to modern consumer preferences concerning marking and certification.

**Example 6.**

There was a laboratory study of washing effectiveness of the composition included in the formulation of the detergent (formulation No. 1) comparing to control detergents (formulations No.2 and No.3) (Table 7).

Table 7. Formulation of liquid dishwashing detergents

| No . | Name of formulation | Ingredients | Naturality, Certification |
|---|---|---|---|
| 1 | Dishwashing detergent with the lipase-free composition | Aqua, Poly(oxy-1,2-ethanediyl), alpha.-sulfo-.omega.-hydroxy-, C10-16-alkyl ethers, sodium salts, Decyl Glucoside, C8-18/18-unsaturated amidopropyl betaine, Glycerin, Sorbitol, Citric Acid, Tetrasodium Glutamate Diacetate, Gossypium Herbaceum Extract, Silver Citrate, Preservatives, Any functional additives. | 96% Vitality Leaf, Ecolabel, Halal |
| 2 | Dishwashing detergent with the lipase-containing composition | Aqua, Poly(oxy-1,2-ethanediyl), alpha.-sulfo-.omega.-hydroxy-, C10-16-alkyl ethers, sodium salts, Decyl Glucoside, C8-18/18-unsaturated amidopropyl betaine, Glycerin, Sorbitol, Lipase, Citric Acid, Tetrasodium Glutamate Diacetate, Gossypium Herbaceum Extract, Silver Citrate, Preservatives, Any functional additives. | 96% Vitality Leaf, Ecolabel, Halal |
| 3 | Alternative dishwashing detergent No. 1 | Aqua, Sodium Laureth Sulfate, Lauramine Oxide, Alcohol, Sodium Chloride, Poly[oxy(methyl-1,2-ethanediyl)], .alpha.-hydro-.omega.-hydroxy- (n=17), Parfum, PEI/PEG/PPG Copolymer, Phenoxyethanol, Sodium Hydroxide, 4-Methylcyclohexane-1,3-diamine, Limonene, Magnesium Sulfate, Dipropylene Glycol, 2-Methylcyclohexane-1,3-diamine, Citronellol, Benzisothiazolinone, Colorant, Methylisothiazolinone. (https://www.info-pg.com/eng/variants/United%20Kingdom?prodForm=507 ) | <70% Absence |
| 4 | Alternative dishwashing detergent No. 2 | Aqua, Sodium Laureth Sulfate, Sodium Lauryl Sarcosinate, Disodium Cocoamphodiacetate, Glycereth-7 Cocoate, Lauryl Glucoside, Glycereth-2 Cocoate, Glycerin, Sodium Chloride, Magnesium sulfate, Citric acid, Nonallergic Parfume with natural oils. (https://synergetic.ru/catalog/sredstva-dlya-mytya-posudy/synergetic-dlya-mytya-posudy-s-aromatom-aloe-0-5l-dozator/) | 95% ICEA |

The base of the dishwashing detergent was prepared in the following way: solubilizers glycerol, sorbitol and the chelating agent glutamate diacetate tetrasodium salt were mixed with water purified during mixing in a common mixer till mixture homogeneity. Also, cotton extract and an aqueous solution of citric acid and silver citrate were added. At the very end there were added an organic acid for pH control and preservatives for ensuring microbiological stability during the storage period. Then alkyl and/or alkyl sulfate and/or alkyl polyethylene glycol sulfate were homogenized with a sufficient quantity of purified water. Then alkylglucoside with the general formula R28-O-[G]p3, a solution of alkyl and/or alkyl sulfate and/or alkyl polyethylene glycol sulfate and biolipase were added. The ingredients were mixed until a homogeneous transparent solution was obtained. After mixing pH of the product was measured and adjusted by adding sodium hydroxide and/or citric acid monohydrate.

The test methods were based on OST 6-15-1662-90 "Household detergents. Methods of determination of washing capacity for different surfaces" and ISO 4198 "Surface active agents - Detergents for hand dishwashing - Guide for comparative testing of performance", GOST 22567.1, GOST 32443, GOST 8420. These tests enabled comprehensive assessment of formulations by parameters of foaming capacity, AS rinsability from tableware surface, detergent viscosity, washing capacity and effectiveness of the dishwashing detergent with simulation of actual conditions.

The method of OST 6-15-1662-90 is that the mass of removed artificial oil and fat soil was determined during certain time with the test detergent in relation to the control detergent by the gravimetric method. Glass plates were selected as sufaces. The glass plates were thoroughly washed, rinsed and dried in a drying oven at the temperature of 120° during 60 minutes and wiped with ethyl alcohol. After preparation the plates were weighed to determine the baseline. Then there was prepared complex oil and fat soiling consisting of grease based on leached industrial oil, lanolin, lecithin emulsifier, egg yolk, flax seed oil (C18:1 oleic acid up to 29%, C18:2 linoleic acid up to 30%, C18:3 linolenoic acid up to 61%), sunflower oil (C16:1 palmitoleic acid up to 0.3%, C18:1 oleic acid up to 39.4%, C18:2 linoleic acid up to 74%, C18:3 linolenoic acid up to 0.3%, C22:1 erucic acid up to 0.3%), C18:1 oleic acid and distilled water at the ratio of 4.1:4.9:1.0:5.1:7.3:2.1:4.5:20. Charges of grease and lanolin were mixed at the room temperature, then heated in a water bath up to 70°C and the emulsifier was added, the mixture was cooled and egg yolk and other ingredients were added. The contaminant was applied on plates with a pipette and left at the room temperature for 30 minutes, then it was transferred to a drying oven and kept at $220\pm5$°C during 8 minutes. Then it was cooled and weighed. The samples were used in pure form. The control was a standard detergent with a similar quantitative composition.

Flax seed oil, sunflower oil and C18:1 oleic acid contain low-melting saturated and/or unsaturated acids and their monoacyl-, diacyl- and triacylglycerides with a different number of bonds with $sp^2$-hybridization having cis- or trans-configuration. The presence of a double bond in the molecule of the substance gives it increased chemical activity - connection of chemical radicals with a double bond and polymerization with opening of a double bond, the greater the specific share of double bonds in fats is, the more prone they are to oxidation. This, in particular, explains oxidation of unsaturated fats with air oxygen, which causes racidification of food oil with the flow of time and drying - formation of a strong film as a result of oxidative polymerization of vegetable oil, for example, flax seed oil and sunflower oil.

To conduct the test each plate was placed into ajar with a lid, capron fabric was put on top and one j ar with a plate was filled with a clean sample of the test detergent, another j ar with plates was filled with 40 ml of the control. The jars were placed into an agitator for liquids and agitated for 5 minutes. After agitation the plates were washed in running water, then rinsed with distilled water and dried in the drying oven at 120°C for an hour, then cooled at the room temperature and weighed.

The obtained data were used to calculate the mass fraction of removed soil. The mass fraction of the removed soil was calculated by the following formula:

$$X = \frac{\text{А}-\text{Б}}{\text{А}-\text{В}} * 100,$$

where

A is the weight of a piece of the plate with the soil before washing with the detergent, g;

Б is the weight of the piece of the plate with the soil after washing with the detergent, g.

B is the weight of the clean plate, g.

The parameter for general assessment of the washing effectiveness and kinetics of cleavage of lipid soils included in the detergent was calculation of % of the washing effectiveness determined by the lab technician based on the test results. The indicator of the mass fraction of the removed soil makes it possible to assess washing effectiveness with respect to complex lipid-based soils with daily use of the composition included in the dishwashing detergent. The general positive trend of use of the dishwashing detergent with the claimed composition is improvement of effectiveness of the dishwashing detergent and cleaner of different surfaces.

Similarly, effectiveness of the detergent was assessed according to ISO 4198, namely, the number of washed plates with the dose of 5 g of detergents per 5 1 of the detergent solution at 45°C and average water hardness of 5.35 mg-eq/l (15 dH) was assessed. 3 g of the model soil consisting of 20% of the fat base (high-melting lard and beef tallow, low-melting butter and sunflower oil with a high content of C18:1 oleic acid up to 39.4% and C18:2 linoleic acid up to 74%, high-fat milk) were applied on each plate. The tableware soil also includes other products (mashed potatoes, flour, eggs, tea, coffee, cocoa, spices, etc.) being the basis of the consumer basket.

For quantitative data, the group arithmetic mean (M), standard deviation (SD) and standard error of the mean (SEM) were calculated. The obtained data were processed by means of MS Excel program. The probability of differences of the mean at different moments of time was determined with the use of the Student's t-test with normal distribution in independent and dependent samplings. The differences were considered to be significant with the level of significance $p < 0.05$.

Results. Based on the results of assessing washing effectiveness of the samples it was established that the test composition included in the liquid dishwashing detergent has strong washing effect with respect to complex lipid and protein soils comparing to other controls not including components of the claimed composition.

At the end of the study marked changes of the assessed parameter of washing effectiveness were observed. According to the parameter dynamics, addition of lipase increased the degree of removal of complex lipid soil based on low-melting saturated and/or unsaturated acids and their monoacyl-, diacyl- and triacylglycerides with different numbers of bonds with $sp^2$-hybridization having cis- or trans-configuration. Specifically, there was synergetic effect between the components of the composition and biolipase in a single formulation as there was increase of washing effectiveness with respect to lipids 1.6 times and not according to the rule of arithmetic additism. Adding as low as 0.0030% of biolipase made it possible to significantly increase the number of clean plates in actual conditions while preserving aerobic biodegradeability and a high content of natural ingredients. Controls did not demonstrate high removal of lipid soils, which speaks of inadequate effectiveness of surfactants for removal of lipid and protein soils in the process of dishwashing, and they also included synthetic surfactants (Table 8).

Table 8. Assessment of washing effectiveness of dishwashing detergent samples

| Test sample | Components of composition | Mass of removed contaminant, % | Number of clean plates, pcs. | Time of removal of burnt-on grease, min |
|---|---|---|---|---|
| No.1.1 | Alkyl polyglucoside 5-15% + Alkyl and/or alkyl sulfate and/or alkyl polyethylene glycol sulfate 5-15% | 97.2±4.0 | 15 | 25 |
| No. 1.2 | Biolipase 0.0030% | 20.48±4.0 | 3 | 25 |
| No.2.2 | Alkyl polyglucoside 5-15% + Alkyl and/or alkyl sulfate and/or alkyl polyethylene glycol sulfate 5-15% + Biolipase 0.0030% | 155.95+4.0 (+58.75%) | 27 (+12 plates) | <10 (2.5 times faster) |
| No. 3 | - | 117.0±4.0 | 32 | 15 |
| No.4 | - | 83.0±4.0 | 11 | >25 |

An additional assessment parameter was the indicator of formulation rinsability from tableware after a wash cycle, namely, the residual quantity of anionic surfactants expressed as sodium dodecylsulfate (Table 8). The formulation rinsability shows the degree of formulation rinsability from tableware used for cooking and eating. The lower the residual quantity of surfactants after washing is, the lower quantity of substances gets into the human organism together with food and the safer the household chemical is for daily use. Use of petrochemical or synthetic surfactants does not make it possible to obtain 0 value owing to high adhesion on tableware surface.

Table 9. Assessment of rinsability of dishwashing detergent samples

| Test sample | Components of composition | Rinsability of formulation from tableware expressed as SDS, mg/dm$^3$ |
|---|---|---|
| No.1.1 | Alkyl polyglucoside 5-15% + Alkyl and/or alkyl sulfate and/or alkyl polyethylene glycol sulfate 5-15% | 0.130 |
| No.1.2 | Biolipase 0.0030% | 0 |
| No.2.2 | Alkyl polyglucoside 5-15% + Alkyl and/or alkyl sulfate and/or alkyl polyethylene glycol sulfate 5-15% + Biolipase 0.0030% | 0.008 |
| No.3 | - | 0.05 |
| No.4 | - | 0.02 |

Based on the results of rinsability, the composition based on natural aerobically biodegradable surfactants and bioenzymes made it possible to obtain a high degree of rinsability from tableware surface comparing to control household chemicals. Combination of components of the composition enables reducing fixation of surfactants on tableware surface 16 times and obtaining complete rinsability of the formulation, which is not characteristic for household chemicals and demonstrates marked synergism of components.

Complex lipid soils based on low-melting saturated and/or unsaturated acids and their monoacyl-, diacyl- and triacylglycerides with different numbers of bonds with $sp^2$-hybridization having cis- or trans-configuration are stubborn in the process of dishwashing owing to fixation and insufficient action of surfactants. A high content of surfactants increases the residual quantity of substances on tableware after washing, which can negatively affect human health.

The composition of the dishwashing detergent based on natural aerobically biodegradable surfactants and biolipase makes it possible to effectively remove soils form tableware surfaces (glass, metal, polymeric, ceramic ones) and hard-to-get areas, thus reducing the quantity of surfactants in household chemicals, ensuring safety for septic tanks and self-contained sewage systems and achieving almost complete rinsablity from tableware. The analysis of the dish-washing detergent with the claimed composition demonstrated marked synergetic effect with respect to soils that are hard to remove using common dishwashing detergents based on surfactants of natural origin. The composition makes it possible to achieve almost complete rinsability with no loss of effectiveness of the detergent, which reduces the load on environment and human health and provides a high percentage of natural ingredients in the formulation of the household chemical for consumers.

**Example 7.**

A laboratory test of washing effectiveness of the composition included in the detergent formulation comparing to substances for removing burnt-on grease was carried out. The base for addition of components was a mixture consisting of purified water, <5% anionic surfactants, non-ionic surfactants <5%, a preservative. The base of the dishwashing detergent was prepared in the following way: preliminarily anionic surfactants were homogenized with a sufficient quantity of purified water while heating at 40-60°C, non-ionic surfactants and a preservative for microbiological stability during the storage life were added. The ingredients were mixed until a homogeneous transparent solution was obtained. After mixing pH of the product was measured and adjusted by adding pH adjusters until the required value was obtained.

The test methods were based on STO 20.41.32-3.13-70864601-2017 "Household cleaning detergents. The method of determination of effectiveness of removing burnt-on fats from stainless steel surface". This test enables assessing effectiveness of the composition by the rate of cleavage of burnt-on grease from a hydrophilic metal surface imitating actual conditions. The test methods were described in detail in Example 6.

Results. Based on the results of assessment of effectiveness and rate of fat cleavage it was established that the test composition included in the all-purpose cleaner has marked washing effect with respect to complex lipid soils based on low-melting saturated and/or unsaturated acids and their monoacyl-, diacyl- and triacylglycerides with different numbers of bonds with $sp^2$-hybridization having cis- or trans-configuration, comparing to controls free of components of the claimed composition and containing other common ingredients (Table 10).

Table 10. Assessment of fat removal effectiveness

| Test sample | Components of composition | Mass of removed fat, % | Time of fat removal, min |
|---|---|---|---|
| Sample No. 1 | no | 75% | 30 |
| Sample No.2 | biolipase 0.006% | 80% | 25 |
| Sample No.3 | biolipase 0.030% | 90% | 15 |
| Sample No.4 | soap <5% | 75% | >30 |
| Sample No.5 | organic solvent <5% | 75% | >30 |

Complex lipid soils based on low-melting saturated and/or unsaturated acids and their monoacyl-, diacyl- and triacylglycerides with different numbers of bonds with $sp^2$-hybridization having cis- or trans-configuration are stubborn in the process of dishwashing owing to fixation and insufficient action of surfactants. Low effectiveness of the detergent results in increased consumption of water and the detergent itself, which is not in line with the trend of sustainable development and ecological friendliness of detergents.

The composition of the dishwashing detergent based on natural aerobically biodegradable surfactants and biolipase enables effective removal of soils from household surfaces (glass, metal, polymeric, ceramic ones) and hard-to-get areas and reduction of time of cleavage of low-melting triglycerides, consumption of water resources and the detergent itself. The analysis of the composition in the formulation of an all-purpose spray revealed marked synergetic effect comparing to known fat removers of non-natural origin.

**Example 8.**

A laboratory analysis of washing effectiveness of the composition included in the formulation of the powder laundry detergent comparing to substances for removal of fat soils and sebum was carried out. The base for addition of components was a mixture consisting of 5-15% zeolites, non-ionic surfactants <5%, anionic surfactants <5%, mineral fillers based on alkaline metal salts silicates, sulfates, carbonates.

The test methods were based on GOST 22567.15-95 "Synthetic detergents. Methods of determination of washing capacity". This test enables assessing effectiveness of the composition by the rate of removal of the pigment/oil soiling EMPA 101 from cotton fabric imitating actual wash conditions. Widely spread recommendations in the territory of EU and RF were selected as test conditions, namely, temperature 40°C, detergent concentration 3.5 g/l per standard washing-machine load and the standard wash cycle (cotton).

Results. Based on the results of estimation of effectiveness and rate of removal of sebum, fat soils it was established that the test composition included in the powder laundry detergent has marked washing effect with respect to complex lipid soils based on low-melting saturated and/or unsaturated acids and their monoacyl-, diacyl- and triacylglycerides with different numbers of bonds with $sp^2$-hybridization having cis- or trans-configuration, comparing to controls containing other components and other common ingredients.

At the end of the analysis marked changes of the assessed indicator of effectiveness of removal of pigment/oil soilings were observed. According to the indicator dynamics, addition of highly-effective biolipase together with the selected components of the composition increased the degree of removal of complex lipid soils based on low-melting saturated and/or unsaturated acids and their monoacyl-, diacyl- and triacylglycerides with different numbers of bonds with $sp^2$-hybridization having cis- or trans-configuration. Combination of the components resulted in the optimal mass fraction of anionic surfactants with low foam height to avoid high fixation of surfactants in fabric fibers and risk of dermatological reaction development. The composition with 0.2% bioliopase increased the washing capacity of the powder laundry detergent by 35% in relation to Sample No.5, which is a sign of synergism between the components. Controls did not demonstrate high removal of lipid soils and sebum, which speaks of inadequate effectiveness of surfactants for removal of lipid soils in the washing process, and they also contained synthetic surfactants (Table 11).

Table 11. Assessment of effectiveness of removal of sebum and oil

| Test sample | Components of composition | Mass fraction of AS, % | Washing effectiveness per 1 wash, % | Foam column height, mm |
|---|---|---|---|---|
| Sample No.1 | Biolipase 0.2% + Sodium C12-C18 alkyl sulfate <5% | 3.6+0.1 | 84+4 | 96+12 |
| Sample No.2 | Lipase <1% + Sodium C10-13 Alkyl Benzenesulfonate 5-15% | 5.4±0.2 | 56+4 | 153+12 |
| Sample No.3 | Lipase <1% + Sodium C10-13 Alkyl Benzenesulfonate 5-15% | 5.5+0.2 | 53+4 | 190+12 |
| Sample No.4 | Sodium C10-13 Alkyl Benzenesulfonate 5-15% | 6.8+0.2 | 77±4 | 146+12 |
| Sample No.5 | Sodium C10-13 Alkyl Benzenesulfonate 5-15% | 1.5 | 49+4 | 138+12 |

An additional parameter of assessment was the indicator of fabric base strength reduction after 25 wash cycles, to analyze the impact of the composition on mechanical and strength characteristics of fabric fibers. The test method is described in GOST 3813-72 "Textile materials. Textile fabrics and piece-articles. Methods for determination of brearing under tension". The lower the strength reduction is, the longer the fabric service life is and the lower the laundry deformation degree in the process of wash with the selected detergent is. Use of petrochemical or synthetic surfactants with additives does not enable achieving a low indicator owing to aggressive impact on natural fibers (Table 12).

Table 12. Assessment of mechanical and strength characteristics of fabric

| Test sample | Components of composition | Fabric base strength reduction after 25 wash cycles in a washing machine, % |
|---|---|---|
| Sample No.1 | Biolipase 0.2% + Sodium C12-C18 alkyl sulfate <5% | 6±1 |
| Sample No.2 | Lipase <1% + Sodium C10-13 Alkyl Benzenesulfonate 5-15% | 25±1 |
| Sample No.3 | Sodium C10-13 Alkyl Benzenesulfonate 5-15% | 45±1 |
| Sample No.4 | Sodium C10-13 Alkyl Benzenesulfonate 5-15% | 14±1 |

According to the test results, the composition based on natural aerobically biodegradable surfactants and biolipase made it possible to achieve a low degree of fabric wear and fiber strength changes comparing to control samples with other formulations. Combination of the components of the composition enables increasing fabric resistance to wear 7.5 times in relation to Sample No.3 and achieving almost complete strength preservation, which is not characteristic for household chemicals and demonstrates marked synergism of components. In the process of 25 wash cycles in a washing machine without detergents strength reduced from 2 to 5 owing to mechanical operation of the washing machine and water pressure, therefore the selected composition included in the formulation of the powder laundry detergent has almost no impact on fabric fibers.

Complex lipid soils based on low-melting saturated and/or unsaturated acids and their monoacyl-, diacyl- and triacylglycerides with different numbers of bonds with $sp^2$-hybridization having cis- or trans-configuration are stubborn in the process of laundry owing to fixation in hard-to-get areas, formation of "biofilms" of sebum and insufficient action of surfactants. A high content of surfactants increases residual quantity of substances on fabric fibers, forms a great amount of foam and greatly reduces fabric resistance to wear, which can negatively affect the environment, increases consumption of water resources and reduces laundry effectiveness.

The composition of the laundry detergent based on natural aerobically biodegradable surfactants and biolipase enables effective removal of soils from the surfaces and hard-to-get areas of fabrics, thus reducing the total quantity of surfactants in household chemicals, ensuring safety for people and achieving almost complete removal of pigment and oil soilings from fabric.

**Example 9.**

A laboratory analysis of washing effectiveness of the composition included in the formulation of the liquid laundry detergent comparing to substances for removal of fat soils, sebum, oil was carried out. The base for addition of components was a mixture consisting of <5% anionic surfactants, 5-15% non-ionic surfactants, amphoteric surfactants, a complexing agent, a preservative and pH adjusters. The base of the detergent was prepared in the following way: the complexing agent was mixed with purified water in a common mixer until the mixture became homogeneous. First, anionic surfactants were homogenized with a sufficient amount of purified water while heating at 40-60°C. Then non-ionic surfactants and an anionic surfactant solution were added. At the very end there was added an organic acid for pH control, a preservative for ensuring microbiological stability during the storage period. The ingredients were mixed until a homogeneous transparent solution was obtained. After mixing pH of the product was measured and adjusted by adding sodium hydroxide until the required value was obtained.

The test method was based on the widely recognized method A.I. S.E. and American standard ASTM D 4265-14. This test enables assessing effectiveness of the composition by the change of the Solar Reflectance Index (SRI) with respect to different soils and stains. Widely spread recommendations in the territory of EU and RF were selected as test conditions, namely, temperature 40°C, water hardness 3.9 mg-eq/l, detergent concentration 60 g per standard washing-machine load and the standard wash cycle (cotton). The selected soilings were EMPA 101 (olive oil/soot), EMPA 106 (mineral oil/soot), EMPA 118 (sebum/pigment), EMPA 116 (blood/milk/ink).

SRI is a quantitative index of removal of soilings and stains in s.u. The values are based on indices of whiteness L and color a,b and are calculated mathematically by a special formula. The method has numerous advantages, specifically, high precision, reproducibility, low error (under 5%), it considers the color of laundry and its change in the wash process, different fabric types and it is the closest to actual visual perception and it also levels off action of optical colorants widely used in formulations of laundry detergents. The effective and statistically significant difference is 2 and more units. Based on practical experience, 1 unit corresponds to 5-10% of the washing effectiveness and greatly contributes to the formulation.

Results. Based on the results of assessment of effectiveness and rate of removal of sebum, fat soils it was established that the test composition included in the liquid laundry detergent has marked washing effect with respect to complex lipid soils based on low-melting saturated and/or unsaturated acids and their monoacyl-, diacyl- and triacylglycerides with different numbers of bonds with $sp^2$-hybridization having cis- or trans-configuration comparing to controls containing other components and other common ingredients.

At the end of the analysis marked changes of the assessed indicator of effectiveness of removal of pigment/oil soilings were observed. According to the indicator dynamics, addition of highly-effective biolipase together with the selected components of the composition increased the degree of removal of complex lipid soils based on low-melting saturated and/or unsaturated acids and their monoacyl-, diacyl- and triacylglycerides with different numbers of bonds with $sp^2$-hybridization having cis- or trans-configuration. Combination of the components resulted in a rise of SRI index. The composition with 0.018-0.024% biolipase increased SRI index by more than 2 units for all stains, which is a sign of synergism between the components and a beneficial result, increase of effectiveness by more than 20% according to the method of GOST 22567.15-95. Sample No.1 free of lipase did not demonstrate high removal of lipid soils and sebum, which speaks of insufficient effectiveness of surfactants for removal of lipid soils in the laundry process (Table 13).

Table 13. Assessment of effectiveness of removal of oil and fat stains

| Test sample | Components of composition | SRI index | | | |
|---|---|---|---|---|---|
| | | EMPA 101 | EMPA 106 | EMPA 112 | EMPA 118 |
| | | oleic acid | motor oil | cocoa with mik | sebum |
| Sample No. 1 Gel | Alkyl polyglucoside 5-15% + Alkyl and/or alkyl sulfate and/or alkyl polyethylene glycol sulfate 5-15% | 58.8 | 73.3 | 75.9 | 85.7 |
| Sample No.2 Gel | Alkyl polyglucoside 5-15% + Alkyl and/or alkyl sulfate and/or alkyl polyethylene glycol sulfate 5-15% + Biolipase 0.018% | 60.1* (+1.3 SRI u.) | 74.5* (+1.2 SRI u.) | 80.2** (+4.3 SRI u.) | 88.8** (+3.1 SRI u.) |
| Sample No.3 Gel | Alkyl polyglucoside 5-15% + Alkyl and/or alkyl sulfate and/or alkyl polyethylene glycol sulfate 5-15% + Biolipase 0.024% | 61.5** (+2.7 SRI u.) | 76,4** (+3.1 SRI u.) | 81.2** (+5.3 SRI u.) | 89.3** (+3.6 SRI u.) |
| *average statistically significant difference (p<0.05), increase of standard washing effectiveness by 10-20% **best statistically significant difference (p<0.05), increase of standard washing effectiveness by 20-40% | | | | | |

Complex lipid soils based on low-melting saturated and/or unsaturated acids and their monoacyl-, diacyl- and triacylglycerides with different numbers of bonds with $sp^2$-hybridization having cis- or trans-configuration are stubborn in the process of laundry owing to fixation in hard-to-get areas, formation of "biofilms" of sebum, oxidation of unsaturated fatty acids and insufficient action of surfactants. A high content of surfactants increases residual quantity of substances on fabric fibers, forms a great amount of foam and greatly reduces fabric resistance to wear, which can negatively affect the environment, increases consumption of water resources and reduces laundry effectiveness.

The composition of the laundry detergent based on natural aerobically biodegradable surfactants and biolipase enables effective removal of soils from the surfaces and hard-to-get areas of fabrics, thus reducing the total quantity of surfactants in household chemicals, ensuring safety for people and achieving almost complete removal of pigment and oil soilings from fabric. Removal of oil and fat stains fulfils the hygienic function, prevents excessive absorption of the soil on fabric surface, fast apperance of unpleasant smell on clothes and reduces the number of washes in the long-term prespective. The increase of SRI index demonstrates a high light-refracting capacity of fabric, appearance of whiteness and reduction of yellowish tone on the surfaces of clothes, which speaks of crystal cleanliness without optical whitening agents.

**Claims**

1.  A composition intended for use in a detergent comprising:

    (A) Biolipase obtained by the biotechnology method from a microorganism, where the given biolipase has biological activity over 40 LU/g, pH 4.0-7.0 at 25°C; and

(B) An aerobically readily biodegradable surfactant or a mixture of surfactants selected from alkylglucoside with the general formula: $R^1$-O-[G]$p^1$, where $R^1$ is an alkyl and/or alkenyl group with hydrocarbon chain length from 4 to 22 carbon atoms, G is a saccharide fragment of 5 or 6 carbon atoms, $p^1$ takes a value from 1 to 4;

(C) An aerobically readily biodegradable surfactant or a mixture of surfactants selected from alkylcarboxylate, and/or alkyl sulfate, and/or alkyl polyethylene glycol sulfate with the general formula $R^2$-(O-$CH_2$-$CH_2$-O)$n^1$($SO_3$)$n^2$($CO_2$)$n^3X^1$, where $n^1$ takes a value from 0 to 10 and denotes the number of polyethylene groups, $R^2$ is an alkyl and/or alkenyl group with hydrocarbon chain length from 5 to 22 carbon atoms; $n^2$ takes a value from 0 to 1 and denotes the number of sulfate groups; $n^3$ takes a value from 0 to 1 and denotes the number of carboxyl groups; $X^1$ is an alkaline and/or alkaline earth metal, ammonium, alkylammonium, alkanolammonium, glucoammonium, basic aminoacid cation,

wherein the mass ratio of A, B and C components makes up (0.0003-0.25):(1-25):(0.5-25), respectively.

2. The composition of claim 1 that additionally comprises an anionic surfactant.

3. The composition of claim 2 wherein the anionic surfactant is selected from:

- A salt of a higher fatty acid amide and methylglycine with the general formula $R^3$-C(O)-N(-$CH_3$)-$CH_2$-$CO_2X^2$, where $R^3$ is an alkyl and/or alkenyl group with hydrocarbon chain length from 5 to 21 carbon atoms and $X^2$ is an alkaline and/or alkaline earth metal, ammonium, alkylammonium, alkanolammonium, glucoammonium cation;
- Alkyl polyethylene glycol carboxylate with the general formula: $R^4$-O(-$CH_2$-$CH_2$-O-)$n^4CH_2$-$CO_2X^3$, where $n^4$ takes a value from 1 to 15 and denotes the number of polyethylene glycol groups, $R^4$ is an alkyl and/or alkenyl group with hydrocarbon chain length from 6 to 22 carbon atoms and $X^3$ is an alkaline and/or alkaline earth metal, ammonium, alkylammonium, alkanolammonium, glucoammonium cation;
- A disubstituted salt of 2-sulfocarboxylic acid with the general formula: $R^5$-CH(-$SO_3X^4$)-$CO_2X^4$, where $R^5$ is an alkyl and/or alkenyl group with hydrocarbon chain length from 4 to 20 carbon atoms and $X^4$ is an alkaline and/or alkaline earth metal, ammonium, alkylammonium, alkanolammonium, glucoammonium cation;
- A mono- or disubstituted salt of a higher carboxylic acid amide and glutamic acid with the general formula: $R^6$-C(O)-NH-CH(-$CH_2$-$CH_2$-$CO_2X^5$)-$CO_2X^5$, where $R^6$ is an alkyl and/or alkenyl group with hydrocarbon chain length from 5 to 21 carbon atoms and $X^5$ is an alkaline and/or alkaline earth metal, ammonium, alkylammonium, alkanolammonium, glucoammonium or hydrogen cation;
- An salt of a higher fatty acid amide and glycine with the general formula: $R^7$-C(O)-NH-$CH_2$-$CO_2X^6$, where $R^7$ is an alkyl and/or alkenyl group with hydrocarbon chain length from 5 to 21 carbon atoms and $X^6$ is an alkaline and/or alkaline earth metal, ammonium, alkylammonium, alkanolammonium, glucoammonium cation;
- A salt of a higher fatty acid amide and alanine with the general formula: $R^8$-C(O)-NH-CH(-$CH_3$)-$CO_2X^7$, where $R^8$ is an alkyl and/or alkenyl group with hydrocarbon chain length from 5 to 21 carbon atoms and $X^7$ is an alkaline and/or alkaline earth metal, ammonium, alkylammonium, alkanolammonium, glucoammonium cation;
- A salt of a higher fatty acid amide and 2-aminomethylethanesulfonic acid with the general formula: $R^9$-C(O)-N(-$CH_3$)-$CH_2$-$CH_2$-$SO_3X^8$, where $R^9$ is an alkyl and/or alkenyl group with hydrocarbon chain length from 5 to 21 carbon atoms and $X^8$ is an alkaline and/or alkaline earth metal, ammonium, alkylammonium, alkanolammonium, glucoammonium cation;
- Alkylpolyglucoside hydroxypropylsulfonate with the general formula: $R^{10}$-O-[G]$p^2$-O-$CH_2$-CH(-OH)-$CH_2$-$SO_3X^9$, where $R^{10}$ is an alkyl and/or alkenyl group with hydrocarbon chain length from 6 to 22 carbon atoms, G is a saccharide fragment comprising 5 or 6 carbon atoms, $p^2$ takes a value from 1 to 4 and $X^9$ is an alkaline and/or alkaline earth metal, ammonium, alkylammonium, alkanolammonium, glucoammonium cation;
- Alkylpolyglucoside carboxylate with the general formula: $R^{11}$-O-[G]$p^3$-O-$CH_2$-$CO_2X^{10}$, where $R^{11}$ is an alkyl and/or alkenyl group with hydrocarbon chain length from 6 to 22 carbon atoms, G is a saccharide fragment comprising 5 or 6 carbon atoms, $p^3$ takes a value from 1 to 4 and $X^{10}$ is an alkaline and/or alkaline earth metal, ammonium, alkylammonium, alkanolammonium, glucoammonium cation;
- A salt of a higher fatty acid amide and threonine with the general formula: $R^{12}$-C(O)-NH-CH(-CH(-OH)-$CH_3$)-$CO_2X^{11}$, where $R^{12}$ is an alkyl and/or alkenyl group with hydrocarbon chain length from 5 to 21 carbon atoms and $X^{11}$ is an alkaline and/or alkaline earth metal, ammonium, alkylammonium, alkanolammonium, glucoammonium cation;
- A salt of a higher fatty acid amide and an aminoacid obtained by hydrolysis of proteins from plant raw materials with the general formula: $R^{13}$-C(O)-$AAX^{12}$, where $R^{13}$ is an alkyl and/or alkenyl group with hydrocarbon chain length from 5 to 21 carbon atoms, AA is an aminoacid or peptide obtained during hydrolysis of plant protein and $X^{12}$ is an alkaline and/or alkaline earth metal, ammonium, alkylammonium, alkanolammonium, glucoammonium cation;

or mixtures thereof.

**4.** The composition of claim 1 that additionally comprises an amphoteric surfactant.

**5.** The composition of claim 4 wherein the amphoteric surfactant is selected from:

- A disubstituted salt of acylamphodiacetate with the general formula: $R^{14}$-C(O)-NH-$CH_2$-$CH_2$-N(-$CH_2$-$CO_2X^{13}$)-$CH_2$-$CH_2$-O-$CH_2$-$CO_2X^{13}$, where $R^{14}$ is an alkyl and/or alkenyl group with hydrocarbon chain length from 5 to 21 carbon atoms and $X^{13}$ is an alkaline and/or alkaline earth metal, ammonium, alkylammonium, alkanolammonium, glucoammonium cation;
- An acylamphoacetate salt with the general formula: $R^{15}$-C(O)-NH-$CH_2$-$CH_2$-N(-$CH_2$-$CO_2X^{14}$)-$CH_2$-$CH_2$-OH, where $R^{15}$ is an alkyl and/or alkenyl group with hydrocarbon chain length from 5 to 21 carbon atoms and $X^{14}$ is an alkaline and/or alkaline earth metal, ammonium, alkylammonium, alkanolammonium, glucoammonium cation;
- An alkylamphoacetate salt with the general formula: $R^{16}$-C(=N-$CH_2$-$CH_2$-N((-$CH_2$-$CH_2$-OH)-$CH_2$-$CO_2X^{15}$)-), where $R^{16}$ is an alkyl and/or alkenyl group with hydrocarbon chain length from 5 to 21 carbon atoms and $X^{15}$ is an alkaline and/or alkaline earth metal, ammonium, alkylammonium, alkanolammonium, glucoammonium cation;
- Acylamidoalkylbetaine with the general formula: $R^{17}$-C(O)-NH-$R^{18}$-N((-$CH_3$)$_2$)-$CH_2$-$CO_2$ , where $R^{17}$ is an alkyl and/or alkenyl group with hydrocarbon chain length from 5 to 21 carbon atoms and $R^{18}$ is an alkyl group with hydrocarbon chain length from 1 to 4 carbon atoms;
- Acylamidoalkylhydroxysultaine with the general formula: $R^{19}$-C(O)-NH-$R^{20}$-N(-$CH_3$)$_2$-$CH_2$-CH(-OH)-$CH_2$-$SO_3$, where $R^{19}$ is an alkyl and/or alkenyl group with hydrocarbon chain length from 5 to 21 carbon atoms and $R^{20}$ is an alkyl group with hydrocarbon chain length from 1 to 4 carbon atoms;
- Acylamidoalkylamine oxide with the general formula: $R^{21}$-C(O)-NH-$R^{22}$-N(-$CH_3$)$_2$-O , where $R^{21}$ is an alkyl and/or alkenyl group with hydrocarbon chain length from 5 to 21 carbon atoms and $R^{22}$ is an alkyl group with hydrocarbon chain length from 1 to 4 carbon atoms;
- Alkylbetaine with the general formula: $R^{23}$-N((-$CH_3$)$_2$)-$CH_2$-$CO_2$, where $R^{23}$ is an alkyl and/or alkenyl group with hydrocarbon chain length from 5 to 21 carbon atoms;
- Alkylhydroxysulfate with the general formula: $R^{24}$-N(-$CH_3$)$_2$-$CH_2$-CH(-OH)-$CH_2$-$SO_3$, where $R^{24}$ is an alkyl and/or alkenyl group with hydrocarbon chain length from 6 to 22 carbon atoms;
- Alkylsulfate with the general formula: $R^{25}$-N(-$CH_3$)$_2$-$CH_2$-$CH_2$-$CH_2$-$SO_3$, where $R^{25}$ is an alkyl and/or alkenyl group with hydrocarbon chain length from 6 to 22 carbon atoms;
- Alkylamine oxide with the general formula: $R^{26}$-N(-$CH_3$)$_2$-O, where $R^{26}$ is an alkyl and/or alkenyl group with hydrocarbon chain length from 6 to 22 carbon atoms;

or mixtures thereof.

**6.** The composition of claim 1 that additionally comprises a non-ionic surfactant.

**7.** The composition of claim 6 wherein the non-ionic surfactant is selected from:

- Alkylpolyethyleneglycol with the general formula: $R^{27}$-O(-$CH_2$-$CH_2$-O-)n$^5$H, where n$^5$ takes a value from 2 to 20 and denotes the number of polyethyleneglycol groups and $R^{27}$ is an alkyl and/or alkenyl group with hydrocarbon chain length from 6 to 22 carbon atoms;
- Alkylpolyethylene/propyleneglycol with the general formula: $R^{28}$-O(-$CH_2$-$CH_2$-O-)n$^6$(-CH(-$CH_3$)-$CH_2$-O-)n$^7$H, where n$^6$ takes a value from 2 to 20 and denotes the number of polyethyleneglycol groups, n$^7$ takes a value from 2 to 20 and denotes the number of polypropyleneglycol groups and $R^{28}$ is an alkyl and/or alkenyl group with hydrocarbon chain length from 6 to 22 carbon atoms;
- Dialkylpolyethyleneglycol with the general formula: $R^{29}$-O(-$CH_2$-$CH_2$-O-)n$^8R^{30}$, where n$^8$ takes a value from 2 to 20 and denotes the number of polyethyleneglycol groups, $R^{29}$ is an alkyl and/or alkenyl group with hydrocarbon chain length from 6 to 22 carbon atoms and $R^{30}$ is an alkyl and/or alkenyl group with hydrocarbon chain length from 1 to 12 carbon atoms;
- Dialkylpolyethylene/propyleneglycol with the general formula: $R^{31}$-O(-$CH_2$-$CH_2$-O-)n$^9$(-CH(-$CH_3$)-$CH_2$-O-)n$^{10}$-$R^{32}$, where n$^9$ takes a value from 2 to 20 and denotes the number of polyethyleneglycol groups, n$^{10}$ takes a value from 2 to 20 and denotes the number of polypropyleneglycol

groups , $R^{31}$ is an alkyl and/or alkenyl group with hydrocarbon chain length from 6 to 22 carbon atoms and $R^{32}$ is an alkyl and/or alkenyl group with hydrocarbon chain length from 1 to 12 carbon atoms;

or mixtures thereof.

8. The composition of claim 1 that additionally comprises a dispersion medium for a polysaccharide/solvent.

9. The composition of claim 8 wherein the dispersion medium is selected from:

- An organic alcohol with the general formula: $R^{33}(-OH)s^1$, where $R^{33}$ is an alkyl group with hydrocarbon chain length from 3 to 12 carbon atoms, $s^1$ takes a value from 1 to 12 and denotes the number of hydroxyl groups arranged in the hydrocarbon radical in any order with respect to each other;
- Alkylpolypropyleneglycol with the general formula: $H(-CH(-CH_3)-CH_2-O-)n^{11}R^{34}$, where $n^{11}$ takes a value from 2 to 10 and denotes the number of polypropyleneglycol groups, $R^{34}$ is an alkyl group with hydrocarbon chain length from 1 to 10 carbon atoms;

or mixtures thereof.

10. The composition of claim 1 that additionally comprises a pH adjuster.

11. The composition of claim 10 wherein the pH adjuster is selected from:

- An organic acid with the general formula: $R^{35}(-OH)s^2(-COOH)m^1$, where $R^{35}$ is an alkyl group with hydrocarbon chain length from 1 to 12 carbon atoms, $s^2$ takes a value from 1 to 12 and denotes the number of hydroxyl groups arranged in the hydrocarbon radical in any order with respect to each other, $m^1$ takes a value from 1 to 4 and denotes the number of carboxyl groups arranged in the hydrocarbon radical in any order with respect to each other;
- A solution of a hydroxide of an alkaline and/or alkaline earth metal, ammonia, primary or tertiary alkylamine, primary or tertiary alkanolamine, primary or tertiary glucamine, basic amino acid, citric acid disodium salt, citric acid trisodium salt or mixtures thereof;

or mixtures thereof.

12. The composition of claim 1 that additionally comprises a chelating agent.

13. The composition of claim 12 wherein the chelating agent is selected from:

- A trisodium salt of methylglycinediacetic acid, a tetrasodium salt of glutaminediacetic acid, a trisodium salt of ethylenediamine-(N,N)-disuccinate;
- An organic acid or its salt with an alkaline metal, ammonium, alkylammonium, alkanolammonium, glucoammonium: citric acid, malic acid, tartaric acid, glutaric acid, adipic acid, glucuronic acid, galacturonic acid, galactaric acid, gluconic acid, phytic acid, polytaconic acid, polyacrylic acid, polymethacrylic acid, a copolymer of acrylic and maleic acids, or an organic acid with the general formula $R^{36}(-OH)s^3(-COOH)m^2$, where $R^{36}$ is an alkyl group with hydrocarbon chain length from 1 to 12 carbon atoms, $s^3$ takes a value from 1 to 12 and denotes the number of hydroxyl groups arranged in the hydrocarbon radical in any order with respect to each other, $m^2$ takes a value from 1 to 4 and denotes the number of carboxyl groups arranged in the hydrocarbon radical in any order with respect to each other;

or mixtures thereof.

14. The composition of claim 1 that additionally comprises a soil redeposition inhibitor.

15. The composition of claim 14 wherein the soil redeposition inhibitor is selected from:

- A polysaccharide derivative selected from: a sodium salt of carboxymethylpolysaccharide, hydroxyalkylpolysaccharide, alkylpolysaccharide or mixtures thereof;
- Polyvynilpyrrolidone;
- A water-soluble salt of polyacrylic acid, or polymethacrylic acid, or a copolymer of akrylic/methacrylic, or maleic acid;

or mixtures thereof.

16. The composition of claim 1 that additionally comprises an anti-foaming agent.

17. The composition of claim 16 wherein the anti-foaming agent is selected from:

- A higher carboxylic acid with the general formula: $R^{37}\text{-}CO_2H$, where $R^{37}$ is an alkyl and/or alkenyl group with hydrocarbon chain length from 5 to 21 carbon atoms;
- A higher carboxylic alcohol with the general formula: $R^{38}\text{-}COH$, where $R^{38}$ is an alkyl and/or alkenyl group with hydrocarbon chain length from 5 to 21 carbon atoms;
- A higher carboxylic alcohol ether with the general formula: $R^{39}\text{-}O\text{-}R^{40}$, where $R^{39}$ and $R^{40}$ independently represent an alkyl and/or alkenyl group with hydrocarbon chain length from 4 to 22 carbon atoms;
- A bisamide of alkyldiamine and a higher carboxylic acid with the general formula: $R^{41}\text{-}C(O)\text{-}NH\text{-}R^{42}\text{-}NH\text{-}C(O)\text{-}R^{43}$, where $R^{41}$ and $R^{43}$ independently represent an alkyl and/or alkenyl group with hydrocarbon chain length from 5 to 21 carbon atoms and $R^{42}$ is an alkyl group with hydrocarbon chain length from 1 to 12 carbon atoms;

or mixtures thereof.

18. The composition of claim 1 that additionally comprises a preservative.

19. The composition of claim 18 wherein the preservative is selected from:

- An organic acid or its salt with an alkaline or alkaline-earth metal, ammonium, alkylammonium, alkanolammonium, glucoammonium: benzoic acid, sorbic acid, 4-methoxybenzoic acid, salicylic acid, undecylenic acid;
- An organic alcohol or phenol: phenoxyethanol, benzyl alcohol, caprylyl glycol, ethylhexylglycerol, phenethyl alcohol, 3-methyl-4-isopropylphenol, 2,4-dichlorobenzyl alcohol;
- A biocide of a wide range of action: benzisothiazolinone, methylisothiazolinone, dodecyldipropylene triamine;
- A fungicide: sodium pyrithione, climbazole;

or mixtures thereof.

20. Use of the composition of any of claims 1-19 in a detergent.

21. Use of claim 20 wherein the detergent comprises 0.50-50.25 %wt. of the specified composition.

22. Use of claim 20 in a detergent for cleavage of low-melting acylglycerides of different spatial configurations.

23. Use of claim 20 in a detergent to control foam stability.

24. Use of claim 20 in a detergent for hydrophilization of surfaces.

## INTERNATIONAL SEARCH REPORT

| International application No. |
| --- |
| PCT/RU 2022/000054 |

**A. CLASSIFICATION OF SUBJECT MATTER**

(see supplemental sheet)

According to International Patent Classification (IPC) or to both national classification and IPC

**B. FIELDS SEARCHED**

Minimum documentation searched (classification system followed by classification symbols)

C11D 3/386, 1/66, 1/04, 1/29, 1/83, 1/90, 3/37, 3/43, 3/20, 17/04, C12N 9/14

Documentation searched other than minimum documentation to the extent that such documents are included in the fields searched

Electronic data base consulted during the international search (name of data base and, where practicable, search terms used)

PatSearch (RUPTO Internal), USPTO, PAJ, Espacenet, Information Retrieval System of FIPS

**C. DOCUMENTS CONSIDERED TO BE RELEVANT**

| Category* | Citation of document, with indication, where appropriate, of the relevant passages | Relevant to claim No. |
| --- | --- | --- |
| X | WO 94/26861 A1 (HENKEL KOMMANDITGESELLSCHAFT AUF AKTIEN et al.) 24.11.1994, points 1, 5-7 of claims, page 3, paragraphs 1-3, page 6, paragraph 4, page 7, paragraph 1, page 10, paragraph 3, page 12, paragraph 1, examples | 1,8,10,14-17,20-24 |
| Y | | 2-7, 9, 11-13, 18, 19 |
| X | WO 86/05187 A1 (A.E. STALEY MANUFACTURING COMPANY) 12.09.1986 | 1, 6 |
| Y | KR 2018114717 A1 (LG HOUSEHOLD & HEALTH CARE LTD) 19.10.2018 | 2-7,9,11-13,18-19 |
| Y | US 2001/0044398 A1 (HORST-DIETER SPECKMANN et al.) 22.11.2001 | 7 |

☐ Further documents are listed in the continuation of Box C.  ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 27 July 2022 (27.07.2022) | 18 August 2022 (18.08.2022) |

| Name and mailing address of the ISA/ RU | Authorized officer |
| --- | --- |
| Facsimile No. | Telephone No. |

Form PCT/ISA/210 (second sheet) (July 1998)

**INTERNATIONAL SEARCH REPORT**

International application No.

PCT/RU 2022/000054

A.  CLASSIFICATION OF SUBJECT MATTER

*C11D 3/386* (2006.01)
*C11D 1/66* (2006.01)
*C11D 1/04* (2006.01)
*C11D 1/29* (2006.01)
*C11D 1/83* (2006.01)
*C11D 1/90* (2006.01)
*C11D 3/37* (2006.01)
*C11D 3/43* (2006.01)
*C11D 3/20* (2006.01)
*C11D 17/04* (2006.01)
*C12N 9/14* (2006.01)

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2294756 C1

- WO 2017082961 A1

**Non-patent literature cited in the description**

- **LOFFLER, H. ; HAPPLE, R.** Profile of irritant patch testing with detergents: sodium lauryl sulfate, sodium laureth sulfate and alkyl polyglucoside. *Contact Dermatitis,* 2003, vol. 48 (1), 26-32
- **PROTTEY C ; FERGUSON T.** Factors which determine the skin irritation potential of soaps and detergents. *J Soc Cosmet Chem.,* 1975, vol. 26, 29-46
- **LOFFLER, H. ; HAPPLE, R.** Profile of irritant patch testing with detergents: sodium lauryl sulfate, sodium laureth sulfate and alkyl polyglucoside. *Contact Dermatitis,* 2003, vol. 48 (1), 26-32
- *CHEMICAL ABSTRACTS,* 9001-62-1
- *European Commission Cosmetic Ingredients & Substances Database,* http://ec.europa.eu/growth/tools-databases/cosing
- **H. UHLIG ; E. M. LINSMAIER-BEDNAR.** Industrial enzymes and their applications. *Engineering,* April 1998, 472

- **MAMTA CHAUHAN ; RAJINDER SINGH CHAUHAN ; VIJAY KUMAR GARLAPATI.** Evaluation of a New Lipase from Staphylococcus sp. for Detergent Additive Capability. *BioMed Research International,* vol. 2013, https://doi.org/10.1155/2013/374967. https://www.hindawi.com/journals/bmri/2013/374967
- **MAMTA CHAUHAN ; RAJINDER SINGH CHAUHAN ; VIJAY KUMAR GARLAPATI.** Evaluation of a New Lipase from Staphylococcus sp. for Detergent Additive Capability. *BioMed Research International,* vol. 2013, 6, https://doi.org/10.1155/2013/374967. https://www.hindawi.com/journals/bmri/2013/374967
- **HEMACHANDER, C. ; PUVANAKRISHNAN, R.** Lipase from Ralstonia pickettii as an additive in laundry detergent formulations. *Process Biochemistry,* 2000, vol. 35 (8), 809-814